# EUROPEAN PATENT APPLICATION

(11) **EP 4 144 864 A1**
(43) Date of publication of application: **08.03.2023**
(21) Application number: 21195112.4
(22) Date of filing: 06.09.2021
(51) Int. Cl.: C12Q 1/6883

(54) **AN IN VITRO METHOD FOR DIAGNOSIS FOR ISCHEMIC VASCULAR DISEASES AND RELATED COMPLICATIONS**

(71) Applicant: Prostem Cardiology B.V., 6221 BG Maastricht (NL)
(72) Inventor: Gho, Conradus Ghosal, 6221 BG Maastricht (NL); Duckers, Eric, 6221 BG Maastricht (NL); Gho, Benedictus Constantinus Ghosal, 6221 BG Maastricht (NL)
(74) Representative: Renkema, Jaap

(57) **Abstract**

An *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least two, preferably at least five, preferably at least ten, more preferably at least fourteen, RNA biomarkers from said group are different between the first expression level and the control expression level. An *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least two, preferably at least ten, more preferably at least fourteen, more preferably at least sixteen, more preferably at least eighteen, RNA biomarkers from said group are different between the first expression level and the control expression level.

## Description

### Field of the invention

The invention relates to an *in vitro* method for diagnosis of occurrence and/or assessing severity of ischemic vascular disease (IVD) and/or ischemic vascular disease-related complication (IVDC) in subjects.

### Background of the invention

One of the manifestations of ischemic vascular diseases is coronary artery disease (also referred to as atherosclerotic heart disease, atherosclerotic cardiovascular disease, and coronary heart disease). Coronary artery disease is a disease of the coronary arteries that leads to, or is associated with, the occurrence of several life-threating conditions or complications including myocardial ischemia, angina pectoris, myocardial infarction (also referred to as heart attack), sudden coronary death, coronary death, heart arrhythmia, cardiogenic shock, and others.

Coronary artery disease and related complications are associated or develop as a result of a limitation of the blood flow to the heart as a result of, for instance, a partial or complete blockage of the coronary arteries (e.g. stenosis and/or occlusion of coronary vessels). A common underlying cause for narrowing arteries is atherosclerosis, which is the long-term accumulation of cholesterol-rich plaques in the coronary arteries. Factors which have been associated with atherosclerosis and promote the atherosclerotic plaque development in coronary arteries include (but are not limited to) smoking, high blood pressure, high cholesterol, diabetes or insulin-resistance, aging, sedentary lifestyle, genetic predisposition and others.

Limitation of the blood flow to the heart due to partial or complete blockage of the coronary arteries can cause myocardial ischemia, which is a situation resulting from a lack of oxygen supply to the heart. Depending on the severity of the ischemic event, the lack of blood supply and oxygen may cause a range of manifesting symptoms ranging from relatively mild symptoms, such as chest pain, chest discomfort and shortness of breath (also known as angina pectoris), to relatively more severe complications, such as unstable angina pectoris (chest pain in rest) or myocardial infarction (e.g. heart attack) when the blockage of a coronary artery is severe or occurs suddenly. This leads to loss of cardiomyocytes. Over time, IVD and related complications may cause irreversible damage to the heart such as heart muscle damage accompanied with myocardial scarring without heart muscle regrowth and adverse cardiac remodeling. Under these conditions, the heart can no longer pump blood sufficiently, leading to heart failure with, for instance, symptoms like fluid retention in the lungs, liver, and legs of a patient.

IVD and related complications are the leading cause of death in Western Society and a major cause of hospital admissions. Therefore, IVD and related complication represent a major health concern, which have triggered significant efforts towards finding ways to reduce the mortality and morbidity associated with IVD and related complications. Particularly, efforts have been devoted to devise tests or assays, which help physicians in diagnosing ongoing IVD and related complications or predicting a patient's risk of developing IVD and related complications as early and as reliably as possible.

For the purpose of diagnosing IVD and related complications, physicians largely rely on standard clinical tests, which involve performing a review of a patient's medical history and submitting the patient to standard of care tests and procedures. For the diagnosis of ischemic coronary artery disease these tests and procedures include electrocardiogram (ECG), (stress) echocardiograms, physical stress test, nuclear perfusion scintigraphy scans, coronary angiography, cardiac CT scans, cardiac magnetic resonance (perfusion) imaging or using Holter monitoring, and the like. These tests and procedures suffer from the following drawbacks. First, because some of these tests involve percutaneous measures, they are necessarily invasive and associated with considerable risks and discomfort for the patient. Second, these tests are in general laborious and require specific infrastructure and expertise for analyzing the test results. Therefore, for each patient, the diagnosis of IVD and related complications is complex, time-consuming and cost-inefficient. More importantly, these procedures lack sensitivity and specificity for diagnosing IVD and related complications in general and particularly for diagnosing myocardial or (cerebrovascular and peripheral vascular) vascular ischemia. That is because early ischemic conditions, such as myocardial ischemia can be quiescent or transient and associated with no or little tissue damage. Further, ischemic conditions may also not present with cholesterol-rich plaques or other obstructions. For these reasons, myocardial ischemia may escape detection by these conventional diagnostic methods (e.g. ECG, bicycle stress exercise test, and others). For ischemic vascular disease there are no adequate (or quantitative) detection methods.

Other types of diagnostic blood tests that have been developed over the last decade require the occurrence of irreversible tissue damage (and loss of cells) due to the ischemia leading to the release of intracellular proteins (or substances) into the circulation. One example of such tests consists of diagnostic tests or assays relying on the use of a single pathophysiological marker or blood biomarker (protein or substances circulating in the blood, e.g. in serum, plasma or buffy coat), such as the troponin assay. Troponin is a complex of three regulatory proteins (troponin C, troponin I, and troponin T) that is integral to muscle contraction in skeletal muscle and cardiac muscle, but not in smooth muscle. Troponin has been used as a biomarker of heart diseases, specifically when irreversible (myocardial) tissue damage occurs, for instance during myocardial infarction. This causes the intracellular troponin to be released from the damaged muscle cells into the circulation. Thus, increased levels of circulating troponin have served as an indicator of the extent cardiac muscle cell death/loss. However, this test suffers from a lack of sensitivity and specificity, and often give false-positive results and fails to detect (early) IVD without any significant myocardial damage (such as in for example angina pectoris or silent angina). Hence, the troponin assay can only diagnose patients with a myocardial infarction where extensive ischemia occurred and caused irreversible tissue damage.

Other examples of diagnostic blood tests or assays for detection of ischemic coronary artery disease relying on the use of a single serum biomarker are tests measuring blood levels of heart-type fatty acid-binding protein (H-FABP) or peptidoglycan recognition protein (PGLYRP1) or interleukin-1 receptor-associated kinase 3 (IRAK3) or vanin 3 (VNN3) or creatine kinase-MB isoenzyme (CK-MB) or myoglobin or others. These assays also suffer from the same limitations as the troponin assay, i.e. they can detect tissue necrosis caused by extensive and persistent ischemia but they cannot detect IVD that occurred without necrosis or tissue damage (for instance angina pectoris or silent angina).

Another category of diagnostic blood tests consists of tests relying on the use of a single protein-gene biomarker or a combination of biomarkers. Examples of biomarkers used in such tests include MEF2A, LDLR, ApoB-100, PCSK9, CYP7A1, ARH, LP8, ALOX5AP, PED4D, LTA4, LGALS2, and others (for instance see Table 1 of Robin et al in Journal of the American College of Cardiology, Vol. 50, No.8 (2007), pp. 727-737 or see Table 1 of Gibbons et al in Circulation (2004) Suppl. IV: IV-47-IV-58)). For example, LDLR, APOB, and PCSK are associated with hypercholesterolemia and cholesterol-rich plaque formation and may indirectly be linked to a higher chance of IVD, which, however, may not form or give a late sign of vascular disease.

The biomarkers used in these types of diagnostic tests typically derive from the results of genome wide analysis or linkage analysis, as part of studies investigating the genetic underpinnings of vascular diseases in general. These tests may be used to determine a subject's (predisposed) risk of developing atherosclerotic lesions, but not to identify/diagnose, stratify patients suffering from IVD (i.e. with insufficient blood flow to the heart) or monitor their response to therapy. Moreover, these biomarkers suffer from the same drawbacks mentioned above, i.e. they lack specificity and sensitivity for diagnosing (progression and monitoring of treatment response) IVD and related complications, particularly (but not limited to) myocardial ischemia, cerebrovascular ischemia (such as cerebrovascular ischemic events (CVA, TIA)) and peripheral artery disease, among others. The clinical utility of these tests has proven to be rather limited as these tests failed to translate into intervention programs or treatment plans that improve outcome of patients who tested positive. Furthermore, due to the genetic heterogeneity inherent to different populations and because of sex differences, the results of these tests do not replicate across populations, i.e. the tests do not identify individuals at risk or already suffering from IVD and related complications in all populations.

Another major drawback associated with the diagnostic tests discussed above is the lack of sex-specific diagnostic tests for IVD and related complications. It is well-recognized that major differences exist in the presentation of symptoms, diagnosis, management and outcomes between men and women with IVD and related complications such as myocardial ischemia. IVD and related complications, particularly myocardial ischemia, are frequently under-diagnosed in women compared to men because the current diagnostic tests or assays are not sensitive or reliable enough to detect sex-related differences. Overall, the atypical clinical manifestation and underdiagnosis of IVD in women calls for more objective and tailored diagnostic tests for both men and women.

Therefore, there is a need for alternative diagnostic tests, which are more reliable and more sensitive in diagnosing IVD and related complications, particularly ongoing IVD, such as myocardial ischemia, particularly during the early stage of myocardial ischemia where no significant tissue damage or loss has occurred. Further, there is a need for alternative diagnostic tests, which rely on the use of biomarkers, which are more proximate to the underlying cause(s) of IVD and related complications or ongoing physical conditions associated with IVD and related complications, and which can serve as surrogate biomarkers or surrogate endpoint markers. Further, there is a need for alternative tests or assays which can be used for diagnosing, detecting the occurrence of, assessing severity of or progression of ischemic vascular diseases (IVD) and related complications (e.g. myocardial ischemia) and/or monitoring IVD and related complications (e.g. myocardial ischemia) over time or in response to treatment in female subjects and male subjects, in a more sensitive and reliable manner as well as in a sex-specific manner.

### Objects of the invention

It is an object of the present invention to provide to an *in vitro* method for diagnosis of occurrence and/or assessing severity of ischemic vascular disease (IVD) and/or ischemic vascular disease-related complication (IVDC) in subjects.

It is a further object of the invention to provide to an *in vitro* method for diagnosis of occurrence and/or assessing severity of ischemic vascular disease (IVD) and/or ischemic vascular disease-related complication (IVDC) in specific for female subjects.

It is a further object of the invention to provide to an *in vitro* method for diagnosis of occurrence and/or assessing severity of ischemic vascular disease (IVD) and/or ischemic vascular disease-related complication (IVDC) in specific for male subjects.

### Summary of the invention

An *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level. An *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_12_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level.

The present inventor surprisingly found that a specific sub-group of the RNA biomarkers expressed in or on CD45-positive cells, isolated from a blood sample taken from a subject can be used as reliable and specific RNA biomarkers for diagnosing, detecting ongoing IVD and/or IVDCs (e.g. myocardial ischemia, particularly in the early stage of myocardial ischemia when tissue damage or loss is minimal, absent or not-detectable using current diagnostic methods), determining the damage caused by or severity of IVD and/or IVDCs and/or monitoring the progression of IVD and/or IVDCs over time in either a female subject or a male subject.

The present inventor found that the RNA biomarkers as taught herein can be reliably detected in a sample from a subject comprising total polynucleotide isolated from CD45+ MNC cells using hybridization probes specifically designed to hybridize to a nucleic acid sequence segment of the RNA biomarkers as taught herein, said probe sets having nucleic acid sequences selected from the nucleic acid sequences of tables 1 to 6.

The present inventor found that that detecting the expression level of at least two, preferably at least five, preferably at least ten, more preferably at least fourteen, of the RNA biomarkers as taught herein, could be used in a sample comprising total polynucleotide isolated from CD45+ MNC cells or biofluids obtained from an subject suffering from IVD and/or IVDCs (i.e. for instance myocardial ischemia) compared to the expression of the same RNA biomarkers in a sample comprising total polynucleotide isolated from CD45+ MNC cells or biofluids from an subject not suffering from IVD and/or IVDCs (i.e. myocardial ischemia) could be used as a reliable and sensitive method for diagnosing, detecting ongoing IVD and/or IVDCs (e.g. myocardial ischemia), determining induced stress on cells or tissue (such as for example hypoxic stress), potential damage caused by or severity of IVD and/or IVDCs and/or monitoring the progression of IVD and/or IVDCs over time and/or after treatment in female subjects. The present inventor found that detecting the expression level of at least two, preferably at least ten, preferably at least fourteen, more preferably at least sixteen, more preferably at least eighteen or more of the RNA biomarkers as taught herein of the RNA biomarkers as taught herein, in a sample comprising total polynucleotide isolated from CD45+ MNC cells or biofluids obtained from an subject suffering from IVD and/or IVDCs (i.e. for instance myocardial ischemia) compared to the expression of the same RNA biomarkers in a sample comprising total polynucleotide isolated from CD45+ MNC cells or biofluids from an subject not suffering from IVD and/or IVDCs (i.e. myocardial ischemia) could be used as a reliable and sensitive method for diagnosing, detecting ongoing IVD and/or IVDCs (e.g. myocardial ischemia), determining induced stress on cells or tissue (such as for example hypoxic stress), potential damage caused by or severity of IVD and/or IVDCs and/or monitoring the progression of IVD and/or IVDCs over time and/or after treatment in male subjects. The present inventor found that compared to traditional diagnostic method and RNA biomarkers, the RNA biomarkers as taught herein represent more reliable and sensitive set of RNA biomarkers for the purposes mentioned herein.

Further, the present inventor has found that the RNA biomarkers as taught herein can be divided into two distinct (i.e. non-overlapping) groups of RNA biomarkers, which are referred to herein as sex-specific RNA biomarkers'. The sex-specific RNA biomarkers as taught herein can be used for diagnosing, detecting ongoing IVD and/or IVDCs (e.g. myocardial ischemia), determining induced stress on cells or tissue (such as for example hypoxic stress or mechanical stress), potential damage caused by or severity of IVD and/or IVDC, and/or monitoring the progression of IVD and/or IVDC over time and/or after treatment in each sex (female and male), separately.

The female-specific RNA biomarkers can be detected in a sample, under stringent hybridization conditions, using hybridization probes having a nucleic acid sequence selected from the nucleic acid sequences of tables 1, 2 and 3 while the male-specific RNA biomarkers can be detected in a sample, under stringent hybridization conditions known in the art and indicated by manufacturers (such as for example Affymetrix Human Gene 2.1 ST array), using hybridization probes having a nucleic acid sequence selected from the nucleic acid sequences of tables 4, 5 and 6.

Without being bound to any theories, the RNA biomarkers and methods of the present inventions are believed to be more reliable and more sensitive than conventional RNA biomarkers used in methods for diagnosing, detecting ongoing IVD and/or IVDCs (e.g. myocardial ischemia), determining induced stress on cells or tissue (such as for example hypoxic stress), potential damage caused by or severity of IVD and/or IVDC, and/or monitoring the progression of IVD and/or IVDC over time and/or after treatment in each sex separately because of the nature of the cells from which the RNA biomarkers as taught herein derive, i.e. CD45+ MNC cells. Since CD45+ MNC cells are involved in angiogenesis (formation of new blood vessels from existing ones) and since compensatory angiogenesis is known to occur in tissue undergoing ischemia, the RNA biomarkers of the invention are believed to be more proximate to the underlying cause(s) of IVD and/or IVDCs or ongoing physical conditions associated with IVD and/or IVDCs. RNA biomarkers can be detected in CD45+ MNC cells, as well as in samples that contain CD45+ MNC cells or in biofluids in which the relevant RNA biomarkers have been secreted (from the CD45+ MNC cells).

For instance, the present inventor found that the RNA biomarkers of the invention were more reliable and more sensitive for diagnosing, detecting ongoing IVD and/or IVDCs (particularly the early stage of myocardial ischemia, where tissue damage or loss is minimal or absent), determining induced stress on cells or tissue (such as for example hypoxic stress), potential damage caused by or severity of IVD and/or IVDCs (particularly myocardial ischemia), and/or monitoring the progression of IVD and/or IVDCs (particularly myocardial ischemia) over time and/or after treatment in each sex (female and male) separately, than conventional diagnostic tests and RNA biomarkers used for the same purposes.

### Detailed description of the invention

### Definitions

The term 'ischemic vascular diseases' (abbreviated as IVD) as used herein refers to a disease of the coronary, cerebrovascular or peripheral arteries, where the arteries become damaged, obstructed, vasospastic or dysfunctional. Damaged or diseased coronary arteries can cause (an absolute or relative) reduced blood flow to the organ (for instance (but not limited to) the heart or brain, due to a partial or complete blockage of the arteries (for instance atherosclerotic stenosis and/or occlusion of coronary vessels). A common underlying cause for blocked arteries is atherosclerosis, which is the long-term accumulation of cholesterol-rich plaques in the arteries. Progression of artherosclerosis is accelerated in patients by factors such as smoking, high blood pressure, high cholesterol, diabetes or insulin resistance, sedentary lifestyle, genetic predisposition and others. But in 30% of men and 40-60% of women "non-obstructive" coronary artery disease is found, which does not present with cholesterol-rich plaques or other obstructions. For instance, limitation of the blood flow to the heart or partial or complete blockage of the coronary arteries may cause myocardial ischemia, which is a situation where myocardial cells lack sufficient supply of oxygen by the coronary arteries. Ischemic vascular diseases comprise, but is not limited to, 'coronary artery disease', 'atherosclerotic heart disease', 'atherosclerotic vascular disease', 'coronary heart disease', cerebrovascular ischemic disease, peripheral artery disease, vascular nephropathy, diabetic vasculopathy, Buergers disease, to name a few.

The term 'ischemic vascular disease-related complications' (abbreviated as IVDCs) as used herein refers to complications which develop as a consequence or in association with IVD. Depending on the severity of IVD, the lack of blood supply and oxygen may cause a host of IVDCs for instance relatively mild symptoms and complications, such as myocardial ischemia (angina pectoris) and a transient ischemic cerebrovascular attack (TIA) to relatively more severe complications such irreversible tissue damage due to the acute occlusion of vessels (like for instance myocardial infarction, e.g. also known as 'heart attack', or a cerebrovascular accident (CVA)). For instance, myocardial ischemia ((angina pectoris) may manifest itself as or causes 'chest pain', however may also show no apparent chest pain (silent myocardial ischemia). Other manifestations of angina pectoris may typically include shortness of breath, chest pain associated with shoulder, arm, back, neck, or jaw pain and sensation of heartburn, heart arrhythmia, cardiogenic shock, cardiac arrest, and others. Cerebrovascular transient ischemic events may range from a temporary loss of neurological function with loss of vision or speech, whereas CVA may manifest from persistent speech impairment to hemiplegia. Peripheral artery disease may present itself as claudication intermittent (muscle cramps upon exercise and fatigue). Over time, IVD and IVDCs may cause irreversible damage to the organ that they perfuse for instance the heart which may result in progressive heart muscle loss, myocardial scarring without heart muscle regrowth, adverse tissue remodeling and heart failure. Under these conditions, the heart can no longer pump blood sufficiently.

The term 'ischemia', as used herein, refers to an absolute or relative shortage of the blood supply or an inadequate flow of blood to an organ (e.g. heart, brain), body part or tissue. Relative shortage refers to the discrepancy between blood supply (oxygen delivery) and blood request/demand (oxygen consumption by tissue). The restriction in blood supply, generally due to factors in the blood vessels, is most often, but not exclusively, caused by constriction or blockage of the blood vessels by thromboembolism (blood clots) or atherosclerosis (lipid-laden plaques obstructing the lumen of arteries). But in 30% of men and 40-60% of women "non-obstructive" coronary artery disease is found, which does not present with cholesterol-rich plaques or other obstructions. Ischemia result in damage or dysfunction of tissue (e.g. IVD and IVDCs, myocardial ischemia, stroke, transient ischemic attack (TIA) and the like).

The term 'gene' as used herein refers to a sequence of nucleotides in DNA or RNA that encodes the synthesis of a gene product, either RNA or protein.

The term 'exon' as used herein refers to any part of a gene that will encode a part of the final mature RNA or non-coding RNA transcripts produced by that gene after introns have been removed by RNA splicing.

The term 'RNA biomarkers' as used herein refers to a measurable indicator of some biological state or condition or diseases or complications relating to a disease. RNA biomarkers can be any RNA molecule (such as microRNA (miRNA) and long non coding RNA (IncRNA), messenger RNA (mRNA)) circulating in the blood, present in the biofluid, cells or tissue. In an embodiment of the present invention, the RNA biomarkers are present in or on CD45+ circulating mononuclear cells (MNC) isolated from a sample from a subject, or present in blood or any biofluids (for example urine, serum, plasma, buffy coat, stool, saliva, tissue specimen). The RNA biomarkers of the invention may be detected using hybridization probes from a hybridization probe set for each RNA biomarker, having nucleic acid sequences selected from the nucleic acid sequences detailed in tables 1 to 6. These tables contain the complementary DNA sequences that hybridize to the designated claimed RNA biomarkers, which may be in the form of DNA or RNA depending on the test method used to detect the RNA biomarkers. Alternatively, the RNA biomarker or its gene product can be detected using antibodies raised against said RNA biomarker or its gene product.

The terms 'hybridization probe' and 'hybridization probe set' may also be referred to as 'probe' and 'probe set', respectively.

The term 'stringent hybridization conditions' as used herein refers to a process used to identify polynucleotide sequences, which are substantially identical to a given nucleic acid sequence of a probe. The stringency of the hybridization conditions is sequence dependent and will be different in different circumstances. Method for establishing hybridization conditions are known to the person skilled in the art. Further, hybridization conditions are indicated by manufactures, such as for example Affymetrix Exon Microarray Human Gene 2.1 ST. Generally, stringent conditions are selected to be about 5°C lower than the thermal melting point (Tₘ) for the specific sequences at a defined ionic strength and pH. The Tₘ is the temperature (under defined ionic strength and pH) at which 50% of the target sequence hybridizes to a perfectly matched probe. Typically, stringent conditions will be chosen in which the salt (NaCl) concentration is about 0.02 molar at pH 7 and the temperature is at least 60°C. Lowering the salt concentration and/or increasing the temperature increases stringency. Stringent conditions for RNA-DNA hybridizations (Northern blots using a probe) are for example those which include at least one wash in 0.2X SSC at 63°C for 20 min, or equivalent conditions. Stringent conditions for DNA-DNA hybridization (Southern blots using a probe) are for example those which include at least one wash (usually 2) in 0.2X SSC at a temperature of at least 50°C, usually about 55°C, for 20 min, or equivalent conditions. See also Sambrook *et al.* (1989) and Sambrook and Russell (2001).

In an embodiment, 'high stringency' conditions can be provided, for example, by hybridization at 65°C in an aqueous solution containing 6x SSC (20x SSC contains 3.0 M NaCl, 0.3 M Na-citrate, pH 7.0), 5x Denhardt's (100X Denhardt's contains 2% Ficoll, 2% Polyvinyl pyrrolidone, 2% Bovine Serum Albumin), 0.5% sodium dodecyl sulphate (SDS), and 20 µg/ml denatured carrier DNA (single-stranded fish sperm DNA, with an average length of 120 - 3000 nucleotides) as non-specific competitor. Following hybridization, high stringency washing may be done in several steps, with a final wash (about 30 min) at the hybridization temperature in 0.1-0.2× SSC, 0.1% SDS.

In an embodiment, 'moderate stringency' refers to conditions equivalent to hybridization in the above described solution but at about 60-62° C. In that case the final wash is performed at the hybridization temperature in 1x SSC, 0.1% SDS.

In an embodiment, 'low stringency' refers to conditions equivalent to hybridization in the above described solution at about 50-52° C. In that case, the final wash is performed at the hybridization temperature in 2x SSC, 0.1% SDS. See also Sambrook *et al.* (1989) and Sambrook and Russell (2001).

The term 'probe' or 'hybridization probe' (which can be sex-specific, female or male specific) as used herein refers to a fragment of DNA of variable length (usually 15-1000 bases long), which may be labelled (e.g. with radioactivity or fluorescent label or others). Probes are typically used in samples, comprising total polynucleotides (DNA and/or RNA) to detect the presence of polynucleotide sequences that are complementary to the nucleic acid sequence in the probe. The probe thereby hybridizes to a single-stranded nucleic acid (DNA or RNA) base sequence via probe-target base pairing due to complementarity between the probe and target. The labelled probe may be hybridized to the target ssDNA (Southern blotting) or RNA (Northern blotting) immobilized on a membrane or in situ. Female-specific probe sets, probes and RNA biomarkers are listed in tables 1, 2 and 3 while male-specific probe sets, probes and RNA biomarkers are listed in tables 4, 5 and 6.

To detect hybridization of the probe to its polynucleotide sequence, the probe can be tagged (or "labelled") with a molecular marker of either radioactive or fluorescent molecules; commonly used markers are 32P (a radioactive isotope of phosphorus incorporated into the phosphodiester bond in the probe DNA) or biotin-labeling or Digoxigenin, which is a non-radioactive, antibody-based marker, or others. DNA or RNA sequences that have moderate to high sequence similarity to the probe can then be detected by visualizing the hybridized probe via autoradiography or other imaging techniques. Normally, either X-ray pictures are taken of the filter, or the filter is placed under UV light. Detection of sequences with moderate or high similarity depends on how stringent the hybridization conditions were applied — high stringency, such as high hybridization temperature and low salt in hybridization buffers, permits only hybridization between nucleic acid sequences that are highly similar, whereas low stringency, such as lower temperature and high salt, allows hybridization when the sequences are less similar. Hybridization probes used in DNA microarrays refer to DNA covalently attached to an inert surface, such as coated glass slides or gene chips, to which a mobile cDNA target is hybridized. The RNA samples here were detected using a microarray analysis (photolithography ― Affymetrix Exon Microarray Human Gene 2.1 ST, Affymetrix Santa Clara, CA, USA). Alternative methods of detecting and (semi) quantitative analysis of RNA may include (but is not limited to quantitative PCR reaction, Luminex detection). Any future methodology to detect the RNA (or DNA) sequences (and products derived thereof, such as non-coding RNAs and proteins) will be covered by the patent.

The term 'differentially expressed' in samples comprising total polynucleotide isolated from cells, such as for example CD45+ MNC cells, or differentially expressed in or on cells, such as for example CD45+ MNC cells, isolated from a blood or tissue sample from a subject refers to differences in levels or quantity or frequency (incidence of occurrence) of two or more RNA biomarker as taught herein present in said sample taken from a subject as compared to a control subject or a control population (e.g. reference biobank or database). RNA biomarkers may also be detected in any biofluid (for example stool, saliva, urine, serum, blood, plasma, buffy coat or a tissue specimen) or tissue sample. For example, the level of expression of two or more RNA biomarkers as taught herein, detected from a test sample can be found to be elevated/up-regulated or decreased/down-regulated compared to the levels of the same RNA biomarker detected from a control sample taken from control subject. In the present invention, a RNA biomarker which is differentially expressed in a test sample relative to a control sample is a RNA biomarker that is detected at a substantially higher or lower level or in substantially higher or lower amount or quantity in the test sample relative to the control sample. Preferably, the level of expression of the two or more RNA biomarkers (or products thereof) detected in a sample taken from a subject are statistically higher or lower than the levels of the same two or more RNA biomarkers detected in a sample taken from a control subject. It is understood that the subject and the control subject can either be a female subject or a male subject. Preferably, the subject and the control subject are sex-matched.

The term 'subject' or 'patient' as used herein refers to a subject or a patient, suspected or known to have IVD or IVDCs, respectively. The term 'control subject' as used herein refers to a subject assumed to be without IVD or IVDCs or a control population assumed to be without IVD or IVDCs.

The term 'increased expression level' and 'decreased expression level' of two or more RNA biomarkers (and products derived thereof, such as non-coding RNAs and proteins) as used herein refers to a substantially or significantly increased expression level or substantially or significantly decreased expression level of two or more RNA biomarkers (and products derived thereof, such as non-coding RNAs and proteins) as taught herein. For instance, a level of expression in a test sample is increased or decreased when it is at least 5%, such as 10%, 15%, 20%, 25%, 30%, 35%, 40%, 45%, 50% higher or lower, than the corresponding expression level in a control sample.

The term 'test sample' as used herein refers to a sample (e.g. blood sample, any biofluid sample, or cell sample or tissue sample) taken from a subject or a patient who is at risk of suffering or is suffering from a disease or a related condition (e.g. IVD and/or IVDCs). Typically, a test sample derives from an unhealthy subject or patient.

The term 'control sample' as used herein, refers to a sample (e.g. blood sample, biofluids sample or cell sample or tissue sample) taken from a control subject not suffering or who is not at risk of suffering from a disease or a related condition (e.g. IVD and/or IVDCs). Typically, a control sample derives from a healthy subject.

The term 'determining the damage or severity of IVD and/or IVDCs' as used herein refers to assessing the degree of ischemia, damage or induced stress on cells or tissue (such as for example hypoxic stress) by IVD and/or IVDCs or assessing the severity of IVD and/or IVDCs in a female or male subject by detecting, in a sample (for instance in or on CD45+ MNC cells isolated from a blood, biofluids or tissue samples from a subject, or in a sample comprising total polynucleotides isolated from CD45+ MNC cells (or biofluids/blood (e.g. buffy coat, serum or plasma) that previously contained CD45+ MNC cells), the expression levels of the female-specific RNA biomarkers or male-specific RNA biomarkers as taught herein. Quantitation of the levels of RNA biomarkers as taught herein can be expressed or reported either in absolute amount (e.g. µg/ml) or a relative amount (e.g., relative intensity of signals or fold changes, etc.) to a control sample. The levels of expression of the RNA biomarkers as taught herein can be determined by any suitable methods such as northern blotting, nuclease protection assay, *in situ* hybridization, DNA microarray, exon DNA microarray or quantitative RT-PCR, biochemical assays, Luminex technology, chromatographic assays, mass spectrometric assays or hybridization assays using labelled hybridization probes such as those as taught herein), RNA/DNA microarray assay, RNA/DNA Chip assays or immunoassays, ELISA assays. For instance, in an embodiment of the invention, the damage, induced stress on cells or tissue (such as for example hypoxic stress) or ischemia caused by, or the severity of IVD and/or IVDCs may be determined by detecting how divergent the expression level of two or more RNA biomarkers in a test sample is from the expression level of a control value or the expression level of the same RNA biomarker(s) in a control sample (or control population).

The term "angiogenesis" as used herein refers to the formation of blood vessels from existing ones. Vasculogenesis and angiogenesis both occur in adulthood and embryonic development.

The term 'about', as used herein indicates a range of normal tolerance in the art, for example within 2 standard deviations of the mean. The term 'about' can be understood as encompassing values that deviate at most 10%, 9%, 8%, 7%, 6%, 5%, 4%, 3%, 2%, 1%, 0.5%, 0.1%, 0.05%, or 0.01% of the indicated value.

The term 'comprising' or 'to comprise' and their conjugations, as used herein, refer to a situation wherein said terms are used in their non-limiting sense to mean that items following the word are included, but items not specifically mentioned are not excluded. It also encompasses the more limiting verb 'to consist essentially of' and 'to consist of'. Reference to an element by the indefinite article 'a' or 'an' does not exclude the possibility that more than one of the elements is present, unless the context clearly requires that there be one and only one of the elements. The indefinite article 'a' or 'an' thus usually means 'at least one'.

Circulating Mononuclear Cells (MNC cells) have been shown to be involved in vessel formation and maintenance in development and adult mammals (angiogenesis and vascular homeostasis). MNC cells can be isolated from tissue (for instance, the bone marrow, myocardial tissue) or from a blood or biofluid sample obtained from a subject (e.g. for instance from the mononuclear cell fraction of blood, plasma, saliva, urine, stool sample) using standards cell isolation protocol including, but not limited to Ficoll isolation, immune separation using anti-MNC antibodies. In adulthood, MNC cells are detected in the blood of healthy individuals. However, the amount of MNC cells substantially increases in individuals undergoing or having vascular injury or other diseases or conditions causing vasculogenesis (i.e. formation of new blood vessels) and angiogenesis (i.e. formation of blood vessels from existing ones). For example, CD45+ and/or CD14+ MNC cells have been shown to contribute to new vessel formation (Bastiaansen et al 2014 and Bot et al 2020).

Samples can be taken preferably from CD45+ MNC cells isolated from tissue/blood or biofluid samples, but can also be derived from samples without these relevant cells (since the relevant RNA biomarkers have been secreted (from the cells) into the sample).

CD45+ MNC cells are involved in vasculogenesis and vascular homeostasis (maintenance of existing blood vessels) during embryogenesis and adulthood. CD45+ MNC cells can be isolated from tissue (for instance, the bone marrow, myocardial tissue or tumor) or from a blood or biofluid sample obtained from a subject (e.g. for instance from the mononuclear cell fraction of blood, plasma, buffy coat, saliva, urine, serum, blood, stool sample or a tissue specimen) using a standards cell isolation protocol including, but not limited to, immune separation using anti-CD45 and / or anti-CD14 antibodies. In adulthood, the amount of CD45+ MNC cells substantially increases in individuals undergoing or having vascular injury or other diseases or conditions causing angiogenesis (i.e. formation of blood vessels from existing ones in adult subjects).

In a first aspect the invention provides an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level.

Tables 1 2, and 3 are shown below. Table 1 shows the names of the RNA biomarkers identified using the probes in the probe sets (Probe Set ID), further the chromosomes and strands these are found on, for the female subjects. Table 2 details for the corresponding Probe Set IDs, the Exon_ID, the start and end of the exon and the sequences of the probes within each probe set for the female subjects are shown. Table 3 details the corresponding Probe Set IDs, the Exon_ID, the start and end of the exon and the sequences of the probes within the optimal 14 probe set for the female subjects are shown.

**Table 1. RNA biomarkers, which expression is decreased or increased in a sample comprising total polynucleotides isolated from CD45-positive cells (or biofluids) from a female subject, and corresponding chromosomes, strands and probe set (Probe Set ID) used to identify said female specific RNA biomarkers.**

| **IDENTIFIED RNA BIOMARKER** | **CHROMOSOME** | **STRAND** | **PROBE SET ID** |
|---|---|---|---|
| AK125911 // MMP11 /// | chr22 | + | 16928030 |
| ENST00000465385 // MMP11 /// | | | |
| ENST00000465730 // MMP11 /// | | | |
| ENST00000428253 // MMP11 | | | |
| AK299689 // NKAIN3 /// | chr8 | + | 17069388 |
| ENST00000523367 // NKAIN3 /// | | | |
| ENST00000545532 // NKAIN3 | | | |
| BC039373 // LPAR6 | chr13 | - | 16779021 |
| ENST00000468156 // TBCB | chr19 | + | 16861376 |
| ENST00000493991 // COL2A1 /// | chr12 | - | 16763818 |
| AL834148 // COL2A1 | | | |
| ENST00000531175 // PTS | chr11 | + | 16731259 |
| NM_001102398 // HNRNPR /// | chr1 | - | 16683280 |
| NM_005826 // HNRNPR /// | | | |
| NM_001102399 // HNRNPR /// | | | |
| NM_001102397 // HNRNPR /// | | | |
| ENST00000374616 // HNRNPR /// | | | |
| ENST00000374612 // HNRNPR /// | | | |
| ENST00000302271 // HNRNPR /// | | | |
| AF000364 // HNRNPR /// BC001449 // | | | |
| HNRNPR /// DQ351905 // HNRNPR /// | | | |
| AK297382 // HNRNPR /// AK297405 // | | | |
| HNRNPR /// AK300029 // HNRNPR /// | | | |
| BX647784 // HNRNPR /// | | | |
| ENST00000478691 // HNRNPR /// | | | |
| ENST00000427764 // HNRNPR /// | | | |
| ENST00000426846 // HNRNPR /// | | | |
| ENST00000463552 // HNRNPR /// | | | |
| ENST00000470941 // HNRNPR /// | | | |
| ENST00000476451 // HNRNPR /// | | | |
| ENST00000491459 // HNRNPR | | | |
| NM_001113490 // AMOT /// NM_133265 // | chrX | - | 17113427 |
| AMOT /// ENST00000304758 // AMOT /// | | | |
| ENST00000371959 // AMOT /// | | | |
| ENST00000371962 // AMOT /// | | | |
| ENST00000524145 // AMOT /// AF286598 | | | |
| // AMOT /// ENST00000432214 // AMOT /// | | | |
| ENST00000371958 // AMOT | | | |
| NM_001265577 // KIF18B /// | chr17 | - | 16845809 |
| NM_001264573 // KIF18B /// BC144271 // | | | |
| KIF18B /// ENST00000438933 // KIF18B | | | |
| /// ENST00000339151 // KIF18B /// | | | |
| ENST00000376982 // KIF18B | | | |
| NM_015080 // NRXN2 /// NM_138732 // | chr11 | - | 16739924 |
| NRXN2 /// ENST00000377551 // NRXN2 | | | |
| /// ENST00000377559 // NRXN2 /// | | | |
| ENST00000265459 // NRXN2 /// | | | |
| AB035266 // NRXN2 /// | | | |
| ENST00000345863 // NRXN2 | | | |
| NM_015690 // STK36 /// NM_001243313 // | chr2 | + | 16891029 |
| STK36 /// ENST00000295709 // STK36 /// | | | |
| ENST00000392105 // STK36 /// | | | |
| ENST00000440309 // STK36 /// AF200815 | | | |
| // STK36 /// BC026158 // STK36 /// | | | |
| ENST00000392106 // STK36 /// | | | |
| ENST00000486644 // STK36 /// | | | |
| ENST00000473681 // STK36 /// | | | |
| ENST00000431040 // STK36 | | | |
| NM_016328 // GTF2IRD1 /// NM_005685 | chr7 | + | 17047164 |
| // GTF21RD1 /// NM_001199207 // | | | |
| GTF21RD1 /// ENST00000265755 // | | | |
| GTF21RD1 /// ENST00000424337 // | | | |
| GTF21RD1 /// ENST00000574416 // | | | |
| GTF21RD1 /// ENST00000573543 // | | | |
| GTF21RD1 /// ENST00000455841 // | | | |
| GTF21RD1 /// ENST00000573381 // | | | |
| GTF21RD1 /// AF151354 // GTF21RD1 /// | | | |
| AF104923 // GTF21RD1 /// AF156489 // | | | |
| GTF2IRD1 /// AY648295 // GTF2IRD1 /// | | | |
| AF118270 // GTF21RD1 /// AF089107 // | | | |
| GTF21RD1 /// BC018136 // GTF21RD1 /// | | | |
| AB209389 // GTF21RD1 /// | | | |
| ENST00000476977 // GTF21RD1 /// | | | |
| ENST00000470715 // GTF21RD1 /// | | | |
| ENST00000573869 // GTF21RD1 /// | | | |
| ENST00000572452 // GTF21RD1 | | | |
| NM_144639 // UROC1 /// NM_001165974 | chr3 | - | 16958674 |
| // UROC1 /// ENST00000290868 // | | | |
| UROC1 /// ENST00000383579 // UROC1 | | | |
| /// BC115405 // UROC1 | | | |
| NM_144973 // DENND5B /// | chr12 | - | 16762944 |
| ENST00000389082 // DENND5B /// | | | |
| AK125323 // DENND5B /// | | | |
| ENST00000306833 // DENND5B /// | | | |
| ENST00000536562 // DENND5B | | | |
| NM_198082 // CCDC57 /// | chr17 | - | 16850156 |
| ENST00000389641 // CCDC57 /// | | | |
| ENST00000392347 // CCDC57 /// | | | |
| BC110997 // CCDC57 | | | |
| NM_213596 // FOXN4 /// | chr12 | - | 16769889 |
| ENST00000299162 // FOXN4 /// | | | |
| BC146825 // FOXN4 /// | | | |
| ENST00000355216 // FOXN4 /// | | | |
| ENST00000266856 // FOXN4 /// | | | |
| ENST00000423960 // FOXN4 | | | |
| TCONS_00015170-XLOC_007214 // chr8 | chr8 | - | 17081037 |
| // 100 // 1 // 1 // 0 | | | |
| AK094845 // chr12 // 100 // 1 // 1 // 0 | chr12 | - | 16770033 |
| ENST00000257359 // ADAMTS8 /// | chr11 | - | 16746216 |
| NM_007037 // ADAMTS8 /// AF060153 // | | | |
| ADAMTS8 /// ENST00000414575 // | | | |
| ADAMTS8 | | | |
| ENST00000504429 // NBLA00301 | chr4 | + | 16972455 |
| NM_001142553 // DCHS2 /// | chr4 | - | 16980805 |
| ENST00000443500 // DCHS2 /// | | | |
| NM_001142552 // DCHS2 /// | | | |
| ENST00000339452 // DCHS2 /// | | | |
| ENST00000544161 // DCHS2 | | | |
| NM_003211 // TDG /// ENST00000392872 | chr12 | + | 16756127 |
| // TDG /// AK303631 // TDG /// AK295387 | | | |
| // TDG /// BC037557 // TDG /// | | | |
| ENST00000542926 // TDG /// | | | |
| ENST00000540956 // TDG /// | | | |
| ENST00000542036 // TDG /// AK309792 // | | | |
| TDG /// ENST00000266775 // TDG /// | | | |
| ENST00000544861 // TDG | | | |
| NM_024638 // QTRTD1 /// | chr3 | + | 16944179 |
| NM_001256835 // QTRTD1 /// | | | |
| ENST00000281273 // QTRTD1 /// | | | |
| ENST00000485050 // QTRTD1 /// | | | |
| CR457296 // QTRTD1 /// BC034559 // | | | |
| QTRTD1 /// BC039265 // QTRTD1 /// | | | |
| ENST00000466050 // QTRTD1 /// | | | |
| ENST00000490183 // QTRTD1 /// | | | |
| ENST00000466870 // QTRTD1 /// | | | |
| ENST00000482307 // QTRTD1 /// | | | |
| ENST00000472599 // QTRTD1 | | | |
| NM_033637 // BTRC /// NM_003939 // | chr10 | + | 16708482 |
| BTRC /// NM_001256856 // BTRC /// | | | |
| ENST00000370187 // BTRC /// | | | |
| ENST00000393441 // BTRC /// | | | |
| ENST00000408038 // BTRC /// GU727631 | | | |
| // BTRC /// BC027994 // BTRC /// | | | |
| AF129530 // BTRC /// AK295870 // BTRC | | | |
| /// ENST00000493877 // BTRC | | | |
| NM_033655 // CNTNAP3 /// | chr9 | - | 17094394 |
| NM_001201380 // CNTNAP3B /// | | | |
| ENST00000297668 // CNTNAP3 /// | | | |
| ENST00000377564 // CNTNAP3B /// | | | |
| ENST00000377564 // CNTNAP3 /// | | | |
| AF333769 // CNTNAP3 /// | | | |
| ENST00000477002 // CNTNAP3 /// | | | |
| ENST00000484254 // CNTNAP3B /// | | | |
| ENST00000484254 // CNTNAP3 /// | | | |
| ENST00000467854 // CNTNAP3B /// | | | |
| ENST00000467854 // CNTNAP3 | | | |
| NM_152891 // PRSS33 /// | chr16 | - | 16823203 |
| ENST00000293851 // PRSS33 /// | | | |
| ENST00000570702 // PRSS33 /// | | | |
| BC036846 // PRSS33 | | | |

**Table 2. Probe sets and probes that identifies specific exons in the RNA biomarker, which are used for detecting that expression is decreased or increased of RNA biomarkers in a sample comprising total polynucleotides isolated from CD45-positive cells (or biofluids) from a female subject, corresponding Probe Set IDs the Exon_ID, the start and end of the exon and the nucleic acid sequences of the probes (Nucleic Acid Sequences of Probes) within each probe set for the female subjects.**

| **PROBE SET ID** | **EXON_ID** | **START** | **STOP** | **NUCLEIC ACID SEQUENCES OF PROBES** |
|---|---|---|---|---|
| 16928030 | 5187159 | 24121613 | 24121639 | SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 |
| 17069388 | 5294444 | 63161464 | 63161488 | SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51 |
| 16779021 | 5069104 | 49000747 | 49000882 | SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71 |
| 16861376 | 5134829 | 36612868 | 36613067 | SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102 |
| 16763818 | 5057504 | 48382221 | 48382245 | SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130 |
| 16731259 | 5033908 | 112100845 | 112100894 | SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154 |
| 16683280 | 4997115 | 23645050 | 23645174 | SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186 |
| 17113427 | 5328651 | 112025776 | 112025874 | SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219 |
| 16845809 | 5121729 | 43012694 | 43012718 | SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238 |
| 16739924 | 5039969 | 64415691 | 64415715 | SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255 |
| 16891029 | 5158213 | 219559290 | 219559314 | SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289 |
| 17047164 | 5277350 | 74009348 | 74009372 | SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, SEQ ID NO: 306, SEQ ID NO: 307, SEQ ID NO: 308, SEQ ID NO: 309, SEQ ID NO: 310, SEQ ID NO: 311 |
| 16958674 | 5210076 | 126214905 | 126214951 | SEQ ID NO: 312, SEQ ID NO: 313, SEQ ID NO: 314, SEQ ID NO: 315, SEQ ID NO: 316, SEQ ID NO: 317, SEQ ID NO: 318, SEQ ID NO: 319, SEQ ID NO: 320, SEQ ID NO: 321, SEQ ID NO: 322, SEQ ID NO: 323, SEQ ID NO: 324, SEQ ID NO: 325, SEQ ID NO: 326, SEQ ID NO: 327, SEQ ID NO: 328, SEQ ID NO: 329, SEQ ID NO: 330, SEQ ID NO: 331, SEQ ID NO: 332, SEQ ID NO: 333, SEQ ID NO: 334, SEQ ID NO: 335, SEQ ID NO: 336, SEQ ID NO: 337, SEQ ID NO: 338, SEQ ID NO: 339, SEQ ID NO: 340, SEQ ID NO: 341, SEQ ID NO: 342, SEQ ID NO: 343, SEQ ID NO: 344, SEQ ID NO: 345 |
| 16762944 | 5056811 | 31542322 | 31542346 | SEQ ID NO: 346, SEQ ID NO: 347, SEQ ID NO: 348, SEQ ID NO: 349, SEQ ID NO: 350, SEQ ID NO: 351, SEQ ID NO: 352, SEQ ID NO: 353, SEQ ID NO: 354, SEQ ID NO: 355, SEQ ID NO: 356, SEQ ID NO: 357, SEQ ID NO: 358, SEQ ID NO: 359, SEQ ID NO: 360, SEQ ID NO: 361, SEQ ID NO: 362, SEQ ID NO: 363, SEQ ID NO: 364, SEQ ID NO: 365, SEQ ID NO: 366, SEQ ID NO: 367, SEQ ID NO: 368, SEQ ID NO: 369 |
| 16850156 | 5125247 | 80059559 | 80059583 | SEQ ID NO: 370, SEQ ID NO: 371, SEQ ID NO: 372, SEQ ID NO: 373, SEQ ID NO: 374, SEQ ID NO: 375, SEQ ID NO: 376, SEQ ID NO: 377, SEQ ID NO: 378, SEQ ID NO: 379, SEQ ID NO: 380, SEQ ID NO: 381, SEQ ID NO: 382, SEQ ID NO: 383, SEQ ID NO: 384, SEQ ID NO: 385, SEQ ID NO: 386, SEQ ID NO: 387, SEQ ID NO: 388, SEQ ID NO: 389, SEQ ID NO: 390, SEQ ID NO: 391 |
| 16769889 | 5061882 | 109724524 | 109724548 | SEQ ID NO: 392, SEQ ID NO: 393, SEQ ID NO: 394, SEQ ID NO: 395, SEQ ID NO: 396, SEQ ID NO: 397, SEQ ID NO: 398, SEQ ID NO: 399, SEQ ID NO: 400, SEQ ID NO: 401, SEQ ID NO: 402, SEQ ID NO: 403, SEQ ID NO: 404, SEQ ID NO: 405, SEQ ID NO: 406, SEQ ID NO: 407, SEQ ID NO: 408 |
| 17081037 | 5303509 | 128200292 | 128200316 | SEQ ID NO: 409, SEQ ID NO: 410, SEQ ID NO: 411, SEQ ID NO: 412, SEQ ID NO: 413, SEQ ID NO: 414, SEQ ID NO: 415, SEQ ID NO: 416, SEQ ID NO: 417, SEQ ID NO: 418, SEQ ID NO: 419, SEQ ID NO: 420, SEQ ID NO: 421, SEQ ID NO: 422, SEQ ID NO: 423, SEQ ID NO: 424, SEQ ID NO: 425, SEQ ID NO: 426, SEQ ID NO: 427, SEQ ID NO: 428, SEQ ID NO: 429, SEQ ID NO: 430, SEQ ID NO: 431, SEQ ID NO: 432, SEQ ID NO: 433 |
| 16770033 | 5061983 | 110488818 | 110488842 | SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 450, SEQ ID NO: 451, SEQ ID NO: 452, SEQ ID NO: 453, SEQ ID NO: 454, SEQ ID NO: 455, SEQ ID NO: 456, SEQ ID NO: 457, SEQ ID NO: 458, SEQ ID NO: 459, SEQ ID NO: 460, SEQ ID NO: 461, SEQ ID NO: 462, SEQ ID NO: 463, SEQ ID NO: 464, SEQ ID NO: 465, SEQ ID NO: 466, SEQ ID NO: 467, SEQ ID NO: 468 |
| 16746216 | 5044431 | 130298287 | 130298880 | SEQ ID NO: 469, SEQ ID NO: 470, SEQ ID NO: 471, SEQ ID NO: 472, SEQ ID NO: 473, SEQ ID NO: 474, SEQ ID NO: 475, SEQ ID NO: 476, SEQ ID NO: 477, SEQ ID NO: 478, SEQ ID NO: 479, SEQ ID NO: 480, SEQ ID NO: 481, SEQ ID NO: 482, SEQ ID NO: 483, SEQ ID NO: 484, SEQ ID NO: 485, SEQ ID NO: 486, SEQ ID NO: 487, SEQ ID NO: 488, SEQ ID NO: 489 |
| 16972455 | 5221002 | 174452893 | 174452917 | SEQ ID NO: 490, SEQ ID NO: 491, SEQ ID NO: 492, SEQ ID NO: 493, SEQ ID NO: 494, SEQ ID NO: 495, SEQ ID NO: 496, SEQ ID NO: 497, SEQ ID NO: 498, SEQ ID NO: 499, SEQ ID NO: 500, SEQ ID NO: 501, SEQ ID NO: 502, SEQ ID NO: 503, SEQ ID NO: 504, SEQ ID NO: 505, SEQ ID NO: 506, SEQ ID NO: 507, SEQ ID NO: 508, SEQ ID NO: 509, SEQ ID NO: 510, SEQ ID NO: 511, SEQ ID NO: 512, SEQ ID NO: 513, SEQ ID NO: 514, SEQ ID NO: 515, SEQ ID NO: 516, SEQ ID NO: 517, SEQ ID NO: 518 |
| 16980805 | 5227735 | 155412547 | 155412910 | SEQ ID NO: 519, SEQ ID NO: 520, SEQ ID NO: 521, SEQ ID NO: 522, SEQ ID NO: 523, SEQ ID NO: 524, SEQ ID NO: 525, SEQ ID NO: 526, SEQ ID NO: 527, SEQ ID NO: 528, SEQ ID NO: 529, SEQ ID NO: 530, SEQ ID NO: 531, SEQ ID NO: 532, SEQ ID NO: 533, SEQ ID NO: 534, SEQ ID NO: 535, SEQ ID NO: 536, SEQ ID NO: 537, SEQ ID NO: 538, SEQ ID NO: 539, SEQ ID NO: 540, SEQ ID NO: 541, SEQ ID NO: 542, SEQ ID NO: 543, SEQ ID NO: 544, SEQ ID NO: 545, SEQ ID NO: 546, SEQ ID NO: 547, SEQ ID NO: 548, SEQ ID NO: 549, SEQ ID NO: 550, SEQ ID NO: 551, SEQ ID NO: 552, SEQ ID NO: 553, SEQ ID NO: 554, SEQ ID NO: 555, SEQ ID NO: 556, SEQ ID NO: 557 |
| 16756127 | 5051713 | 104379448 | 104379506 | SEQ ID NO: 558, SEQ ID NO: 559, SEQ ID NO: 560, SEQ ID NO: 561, SEQ ID NO: 562, SEQ ID NO: 563, SEQ ID NO: 564, SEQ ID NO: 565, SEQ ID NO: 566, SEQ ID NO: 567, SEQ ID NO: 568, SEQ ID NO: 569, SEQ ID NO: 570, SEQ ID NO: 571, SEQ ID NO: 572, SEQ ID NO: 573, SEQ ID NO: 574, SEQ ID NO: 575, SEQ ID NO: 576, SEQ ID NO: 577, SEQ ID NO: 578, SEQ ID NO: 579, SEQ ID NO: 580, SEQ ID NO: 581 |
| 16944179 | 5199144 | 113784250 | 113784274 | SEQ ID NO: 582, SEQ ID NO: 583, SEQ ID NO: 584, SEQ ID NO: 585, SEQ ID NO: 586, SEQ ID NO: 587, SEQ ID NO: 588, SEQ ID NO: 589, SEQ ID NO: 590, SEQ ID NO: 591, SEQ ID NO: 592, SEQ ID NO: 593, SEQ ID NO: 594, SEQ ID NO: 595, SEQ ID NO: 596, SEQ ID NO: 597, SEQ ID NO: 598, SEQ ID NO: 599, SEQ ID NO: 600, SEQ ID NO: 601, SEQ ID NO: 602, SEQ ID NO: 603, SEQ ID NO: 604 |
| 16708482 | 5016644 | 103295201 | 103295225 | SEQ ID NO: 605, SEQ ID NO: 606, SEQ ID NO: 607, SEQ ID NO: 608, SEQ ID NO: 609, SEQ ID NO: 610, SEQ ID NO: 611, SEQ ID NO: 612, SEQ ID NO: 613, SEQ ID NO: 614, SEQ ID NO: 615, SEQ ID NO: 616, SEQ ID NO: 617, SEQ ID NO: 618, SEQ ID NO: 619, SEQ ID NO: 620, SEQ ID NO: 621, SEQ ID NO: 622, SEQ ID NO: 623, SEQ ID NO: 624, SEQ ID NO: 625, SEQ ID NO: 626, SEQ ID NO: 627, SEQ ID NO: 628, SEQ ID NO: 629, SEQ ID NO: 630, SEQ ID NO: 631, SEQ ID NO: 632, SEQ ID NO: 633, SEQ ID NO: 634, SEQ ID NO: 635 |
| 17094394 | 5313941 | 47304476 | 47304508 | SEQ ID NO: 636, SEQ ID NO: 637, SEQ ID NO: 638, SEQ ID NO: 639, SEQ ID NO: 640, SEQ ID NO: 641, SEQ ID NO: 642, SEQ ID NO: 643, SEQ ID NO: 644, SEQ ID NO: 645, SEQ ID NO: 646, SEQ ID NO: 647, SEQ ID NO: 648, SEQ ID NO: 649, SEQ ID NO: 650, SEQ ID NO: 651, SEQ ID NO: 652, SEQ ID NO: 653, SEQ ID NO: 654, SEQ ID NO: 655, SEQ ID NO: 656, SEQ ID NO: 657, SEQ ID NO: 658, SEQ ID NO: 659, SEQ ID NO: 660, SEQ ID NO: 661, SEQ ID NO: 662, SEQ ID NO: 663, SEQ ID NO: 664 |
| 16823203 | 5103477 | 2834647 | 2834792 | SEQ ID NO: 665, SEQ ID NO: 666, SEQ ID NO: 667, SEQ ID NO: 668, SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 679, SEQ ID NO: 680, SEQ ID NO: 681, SEQ ID NO: 682, SEQ ID NO: 683, SEQ ID NO: 684, SEQ ID NO: 685 |

**Table 3. Optimal Set of 14 probe sets that identifies specific exons in the RNA biomarker, which are used for detecting that expression is decreased or increased of RNA biomarkers in a sample comprising total polynucleotides isolated from CD45-positive cells (or biofluids) from a female subject, corresponding Probe Set IDs the Exon_ID, the start and end of the exon and the nucleic acid sequences of the probes (Nucleic Acid Sequences of Probes) within each probe set for the female subjects.**

| **PROBE SET ID** | **EXON_ID** | **START** | **STOP** | **NUCLEIC ACID SEQUENCES OF PROBES** |
|---|---|---|---|---|
| 16944179 | 5199144 | 113784250 | 113784274 | SEQ ID NO: 582, SEQ ID NO: 583, SEQ ID NO: 584, SEQ ID NO: 585, SEQ ID NO: 586, SEQ ID NO: 587, SEQ ID NO: 588, SEQ ID NO: 589, SEQ ID NO: 590, SEQ ID NO: 591, SEQ ID NO: 592, SEQ ID NO: 593, SEQ ID NO: 594, SEQ ID NO: 595, SEQ ID NO: 596, SEQ ID NO: 597, SEQ ID NO: 598, SEQ ID NO: 599, SEQ ID NO: 600, SEQ ID NO: 601, SEQ ID NO: 602, SEQ ID NO: 603, SEQ ID NO: 604 |
| 17113427 | 5328651 | 112025776 | 112025874 | SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219 |
| 16762944 | 5056811 | 31542322 | 31542346 | SEQ ID NO: 346, SEQ ID NO: 347, SEQ ID NO: 348, SEQ ID NO: 349, SEQ ID NO: 350, SEQ ID NO: 351, SEQ ID NO: 352, SEQ ID NO: 353, SEQ ID NO: 354, SEQ ID NO: 355, SEQ ID NO: 356, SEQ ID NO: 357, SEQ ID NO: 358, SEQ ID NO: 359, SEQ ID NO: 360, SEQ ID NO: 361, SEQ ID NO: 362, SEQ ID NO: 363, SEQ ID NO: 364, SEQ ID NO: 365, SEQ ID NO: 366, SEQ ID NO: 367, SEQ ID NO: 368, SEQ ID NO: 369 |
| 16972455 | 5221002 | 174452893 | 174452917 | SEQ ID NO: 490, SEQ ID NO: 491, SEQ ID NO: 492, SEQ ID NO: 493, SEQ ID NO: 494, SEQ ID NO: 495, SEQ ID NO: 496, SEQ ID NO: 497, SEQ ID NO: 498, SEQ ID NO: 499, SEQ ID NO: 500, SEQ ID NO: 501, SEQ ID NO: 502, SEQ ID NO: 503, SEQ ID NO: 504, SEQ ID NO: 505, SEQ ID NO: 506, SEQ ID NO: 507, SEQ ID NO: 508, SEQ ID NO: 509, SEQ ID NO: 510, SEQ ID NO: 511, SEQ ID NO: 512, SEQ ID NO: 513, SEQ ID NO: 514, SEQ ID NO: 515, SEQ ID NO: 516, SEQ ID NO: 517, SEQ ID NO: 518 |
| 16756127 | 5051713 | 104379448 | 104379506 | SEQ ID NO: 558, SEQ ID NO: 559, SEQ ID NO: 560, SEQ ID NO: 561, SEQ ID NO: 562, SEQ ID NO: 563, SEQ ID NO: 564, SEQ ID NO: 565, SEQ ID NO: 566, SEQ ID NO: 567, SEQ ID NO: 568, SEQ ID NO: 569, SEQ ID NO: 570, SEQ ID NO: 571, SEQ ID NO: 572, SEQ ID NO: 573, SEQ ID NO: 574, SEQ ID NO: 575, SEQ ID NO: 576, SEQ ID NO: 577, SEQ ID NO: 578, SEQ ID NO: 579, SEQ ID NO: 580, SEQ ID NO: 581 |
| 16763818 | 5057504 | 48382221 | 48382245 | SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130 |
| 16823203 | 5103477 | 2834647 | 2834792 | SEQ ID NO: 665, SEQ ID NO: 666, SEQ ID NO: 667, SEQ ID NO: 668, SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 679, SEQ ID NO: 680, SEQ ID NO: 681, SEQ ID NO: 682, SEQ ID NO: 683, SEQ ID NO: 684, SEQ ID NO: 685 |
| 16708482 | 5016644 | 103295201 | 103295225 | SEQ ID NO: 605, SEQ ID NO: 606, SEQ ID NO: 607, SEQ ID NO: 608, SEQ ID NO: 609, SEQ ID NO: 610, SEQ ID NO: 611, SEQ ID NO: 612, SEQ ID NO: 613, SEQ ID NO: 614, SEQ ID NO: 615, SEQ ID NO: 616, SEQ ID NO: 617, SEQ ID NO: 618, SEQ ID NO: 619, SEQ ID NO: 620, SEQ ID NO: 621, SEQ ID NO: 622, SEQ ID NO: 623, SEQ ID NO: 624, SEQ ID NO: 625, SEQ ID NO: 626, SEQ ID NO: 627, SEQ ID NO: 628, SEQ ID NO: 629, SEQ ID NO: 630, SEQ ID NO: 631, SEQ ID NO: 632, SEQ ID NO: 633, SEQ ID NO: 634, SEQ ID NO: 635 |
| 16980805 | 5227735 | 15541254 7 | 15541291 0 | SEQ ID NO: 519, SEQ ID NO: 520, SEQ ID NO: 521, SEQ ID NO: 522, SEQ ID NO: 523, SEQ ID NO: 524, SEQ ID NO: 525, SEQ ID NO: 526, SEQ ID NO: 527, SEQ ID NO: 528, SEQ ID NO: 529, SEQ ID NO: 530, SEQ ID NO: 531, SEQ ID NO: 532, SEQ ID NO: 533, SEQ ID NO: 534, SEQ ID NO: 535, SEQ ID NO: 536, SEQ ID NO: 537, SEQ ID NO: 538, SEQ ID NO: 539, SEQ ID NO: 540, SEQ ID NO: 541, SEQ ID NO: 542, SEQ ID NO: 543, SEQ ID NO: 544, SEQ ID NO: 545, SEQ ID NO: 546, SEQ ID NO: 547, SEQ ID NO: 548, SEQ ID NO: 549, SEQ ID NO: 550, SEQ ID NO: 551, SEQ ID NO: 552, SEQ ID NO: 553, SEQ ID NO: 554, SEQ ID NO: 555, SEQ ID NO: 556, SEQ ID NO: 557 |
| 16779021 | 5069104 | 49000747 | 49000882 | SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71 |
| 16770033 | 5061983 | 110488818 | 11048884 2 | SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 450, SEQ ID NO: 451, SEQ ID NO: 452, SEQ ID NO: 453, SEQ ID NO: 454, SEQ ID NO: 455, SEQ ID NO: 456, SEQ ID NO: 457, SEQ ID NO: 458, SEQ ID NO: 459, SEQ ID NO: 460, SEQ ID NO: 461, SEQ ID NO: 462, SEQ ID NO: 463, SEQ ID NO: 464, SEQ ID NO: 465, SEQ ID NO: 466, SEQ ID NO: 467, SEQ ID NO: 468 |
| 17094394 | 5313941 | 47304476 | 47304508 | SEQ ID NO: 636, SEQ ID NO: 637, SEQ ID NO: 638, SEQ ID NO: 639, SEQ ID NO: 640, SEQ ID NO: 641, SEQ ID NO: 642, SEQ ID NO: 643, SEQ ID NO: 644, SEQ ID NO: 645, SEQ ID NO: 646, SEQ ID NO: 647, SEQ ID NO: 648, SEQ ID NO: 649, SEQ ID NO: 650, SEQ ID NO: 651, SEQ ID NO: 652, SEQ ID NO: 653, SEQ ID NO: 654, SEQ ID NO: 655, SEQ ID NO: 656, SEQ ID NO: 657, SEQ ID NO: 658, SEQ ID NO: 659, SEQ ID NO: 660, SEQ ID NO: 661, SEQ ID NO: 662, SEQ ID NO: 663, SEQ ID NO: 664 |
| 16746216 | 5044431 | 130298287 | 130298880 | SEQ ID NO: 469, SEQ ID NO: 470, SEQ ID NO: 471, SEQ ID NO: 472, SEQ ID NO: 473, SEQ ID NO: 474, SEQ ID NO: 475, SEQ ID NO: 476, SEQ ID NO: 477, SEQ ID NO: 478, SEQ ID NO: 479, SEQ ID NO: 480, SEQ ID NO: 481, SEQ ID NO: 482, SEQ ID NO: 483, SEQ ID NO: 484, SEQ ID NO: 485, SEQ ID NO: 486, SEQ ID NO: 487, SEQ ID NO: 488, SEQ ID NO: 489 |
| 16928030 | 5187159 | 24121613 | 24121639 | SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 |

In a second aspect the invention provides an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level.

Tables 4, 5 and 6 are shown below. Table 4 shows the names of the RNA biomarkers identified using the probes in the probe sets (Probe Set ID), further the chromosomes and strands these are found on, for the male subjects. Table 5 details for the corresponding Probe Set IDs the Exon_ID, the start and end of the exon and the sequences of the probes within each probe set for the male subjects are shown. Table 6 details the corresponding Probe Set IDs, the Exon_ID, the start and end of the exon and the sequences of the probes within the optimal 16 probe set for the male subjects are shown.

**Table 4. RNA biomarkers, which expression is decreased or increased in a sample comprising total polynucleotides isolated from CD45-positive cells (or biofluids) from a male subject, and corresponding chromosomes, strands and probe set (Probe Set ID) used to identify said male specific RNA biomarkers.**

| **IDENTIFIED RNA BIOMARKER** | **CHROMOSOME** | **STRAND** | **PROBE SET ID** |
|---|---|---|---|
| ENST00000440123 // chr10 // 100 // 12 // 12 // 0 | chr10 | + | 16708531 |
| TCONS_00025321-XLOC_012127 // chr17 // 100//12//11//0 | chr17 | + | 16831561 |
| ENST00000488290 // MTPAP /// AK125739 // LOC729668 | chr10 | - | 16712992 |
| ENST00000430988 // chr2 // 100 // 9 // 9 // 0 | chr2 | - | 16895114 |
| ENST00000440657 // USP12-AS1 | chr13 | + | 16773468 |
| ProbSetID_17118935 | chrU | + | 17118935 |
| NM_007117 // TRH /// ENST00000302649 // TRH /// BC110515 // TRH | chr3 | + | 16945454 |
| XR_109422 // LOC1 00507049 | chr17 | - | 16848054 |
| ENST00000456944 // LOC100507009 /// XR_108972 // LOC1 00507009 | chr9 | - | 17095583 |
| NM_080717 // TBX5 /// ENST00000349716 // TBX5 /// ENST00000310346 // TBX5 /// NR_038440 // TBX5-AS1 /// ENST00000531202 // TBX5-AS1 /// ENST00000532697 // TBX5-AS1 /// ENST00000531024 // TBX5-AS1 | chr12 | + | 16757527 |
| TCONS_I2_00026656-XLOC_I2_013873 // chr7 // 100 // 3 // 3 // 0 | chr7 | - | 17058221 |
| ExonID_5262586 | chr6 | - | 17025653 |
| AK293404 // RGL3 /// ENST00000453604 // RGL3 | chr19 | - | 16868954 |
| ENST00000338315 // MFSD2B /// ENST00000469562 // MFSD2B | chr2 | + | 16877665 |
| ENST00000432646 // CALD1 /// ENST00000443197 // CALD1 | chr7 | + | 17051908 |
| ENST00000518932 // chr8 // 100 // 1 // 1 // 0 | chr8 | - | 17080053 |
| NM_001033551 // TOM1L2 /// NM_001082968 // TOM1L2 /// ENST00000379504 // TOM1L2 /// ENST00000318094 // TOM1L2 /// AK303305 // TOM1L2 /// AK295111 // TOM1L2 /// AK299872 // TOM1L2 /// AK301944 // TOM1L2 /// AK294846 // TOM1 L2 /// BC051650 // TOM1L2 /// ENST00000395739 // TOM1L2 /// ENST00000535933 // TOM1L2 /// ENST00000540946 // TOM1L2 /// ENST00000542206 // TOM1L2 /// ENST00000537091 //TOM1L2 | chr17 | - | 16842003 |
| NM_001105206 // LAMA4 /// NM_002290 // LAMA4 /// NM_001105207 // LAMA4 /// ENST00000230538 // LAMA4 /// ENST00000522006 // LAMA4 /// ENST00000389463 // LAMA4 /// ENST00000424408 // LAMA4 /// BC066552 // LAMA4 /// ENST00000454881 // LAMA4 /// ENST00000521398 // LAMA4 /// ENST00000542588 // LAMA4 /// ENST00000524032 // LAMA4 /// ENST00000423735 // LAMA4 /// ENST00000368639 // LAMA4 | chr6 | - | 17022799 |
| NM_182480 // COQ6 /// ENST00000394026 // COQ6 /// AK296040 // COQ6 /// ENST00000554341 // COQ6 /// ENST00000238709 // COQ6 | chr14 | + | 16786369 |
| ENST00000481363 // SALL4 | chr20 | - | 16920377 |
| NM_138375 // CABLES1 /// ENST00000420687 // CABLES1 /// BC037218 // CABLES1 | chr18 | + | 16851430 |
| NM_005035 // POLRMT /// ENST00000215591 // POLRMT /// BC098387 // POLRMT | chr19 | - | 16866470 |
| ENST00000503919 // SLC23A1 | chr5 | - | 17000583 |
| NM_024807 // TREML2 /// ENST00000483722 // TREML2 /// BC125078 // TREML2 | chr6 | - | 17019044 |
| NR_003697 // SNHG15 /// AK096179 // SNHG15 | chr7 | - | 17057414 |
| NM_000786 // CYP51A1 /// NM_001146152 // CYP51A1 /// ENST00000003100 // CYP51A1 /// ENST00000496998 // CYP51A1 /// ENST00000450723 // CYP51A1 /// U23942 // CYP51A1 /// ENST00000422722 // LRRD1 | chr7 | - | 17059640 |
| ExonID_5299845 | chr8 | - | 17076434 |
| TCONS_00017554-XLOC_008249 // chrX // 100//2//2//0 | chrX | - | 17114699 |

**Table 5. Probe sets and probes that identifies specific exons in the RNA biomarker, which are used for detecting that expression is decreased or increased of RNA biomarkers in a sample comprising total polynucleotides isolated from CD45-positive cells (or biofluids) from a male subject, corresponding Probe Set IDs the Exon_ID, the start and end of the exon and the nucleic acid sequences of the probes (Nucleic Acid Sequences of Probes ) within each probe set for the male subjects.**

| **PROBE SET ID** | **EXON_ID** | **START** | **STOP** | **NUCLEIC ACID SEQUENCES OF PROBES** |
|---|---|---|---|---|
| 16708531 | 5016680 | 103880410 | 103880730 | SEQ ID NO: 686, SEQ ID NO: 687, SEQ ID NO: 688, SEQ ID NO: 689, SEQ ID NO: 690, SEQ ID NO: 691, SEQ ID NO: 692, SEQ ID NO: 693, SEQ ID NO: 694, SEQ ID NO: 695, SEQ ID NO: 696, SEQ ID NO: 697, SEQ ID NO: 698, SEQ ID NO: 699, SEQ ID NO: 700, SEQ ID NO: 701, SEQ ID NO: 702, SEQ ID NO: 703 |
| 16831561 | 5110382 | 16926501 | 16926546 | SEQ ID NO: 704, SEQ ID NO: 705, SEQ ID NO: 706, SEQ ID NO: 707, SEQ ID NO: 708, SEQ ID NO: 709, SEQ ID NO: 710, SEQ ID NO: 711, SEQ ID NO: 712, SEQ ID NO: 713, SEQ ID NO: 714, SEQ ID NO: 715, SEQ ID NO: 716, SEQ ID NO: 717, SEQ ID NO: 718, SEQ ID NO: 719, SEQ ID NO: 720, SEQ ID NO: 721, SEQ ID NO: 722 |
| 16712992 | 5020340 | 30656698 | 30656743 | SEQ ID NO: 723, SEQ ID NO: 724, SEQ ID NO: 725, SEQ ID NO: 726, SEQ ID NO: 727, SEQ ID NO: 728, SEQ ID NO: 729, SEQ ID NO: 730, SEQ ID NO: 731, SEQ ID NO: 732 |
| 16895114 | 5161268 | 23746962 | 23747184 | SEQ ID NO: 733, SEQ ID NO: 734, SEQ ID NO: 735, SEQ ID NO: 736, SEQ ID NO: 737, SEQ ID NO: 738, SEQ ID NO: 739, SEQ ID NO: 740, SEQ ID NO: 741, SEQ ID NO: 742, SEQ ID NO: 743, SEQ ID NO: 744, SEQ ID NO: 745, SEQ ID NO: 746, SEQ ID NO: 747, SEQ ID NO: 748, SEQ ID NO: 749, SEQ ID NO: 750, SEQ ID NO: 751, SEQ ID NO: 752, SEQ ID NO: 753, SEQ ID NO: 754, SEQ ID NO: 755, SEQ ID NO: 756, SEQ ID NO: 757 |
| 16773468 | 5064532 | 27737001 | 27737060 | SEQ ID NO: 758, SEQ ID NO: 759, SEQ ID NO: 760, SEQ ID NO: 761, SEQ ID NO: 762, SEQ ID NO: 763, SEQ ID NO: 764, SEQ ID NO: 765, SEQ ID NO: 766, SEQ ID NO: 767, SEQ ID NO: 768, SEQ ID NO: 769, SEQ ID NO: 770, SEQ ID NO: 771, SEQ ID NO: 772, SEQ ID NO: 773, SEQ ID NO: 774 |
| 17118935 | 0 | 17 | 47 | SEQ ID NO: 775, SEQ ID NO: 776, SEQ ID NO: 777, SEQ ID NO: 778, SEQ ID NO: 779, SEQ ID NO: 780, SEQ ID NO: 781 |
| 16945454 | 5200110 | 129695566 | 129696022 | SEQ ID NO: 782, SEQ ID NO: 783, SEQ ID NO: 784, SEQ ID NO: 785, SEQ ID NO: 786, SEQ ID NO: 787, SEQ ID NO: 788, SEQ ID NO: 789, SEQ ID NO: 790, SEQ ID NO: 791, SEQ ID NO: 792, SEQ ID NO: 793, SEQ ID NO: 794, SEQ ID NO: 795, SEQ ID NO: 796, SEQ ID NO: 797, SEQ ID NO: 798, SEQ ID NO: 799, SEQ ID NO: 800, SEQ ID NO: 801, SEQ ID NO: 802, SEQ ID NO: 803 |
| 16848054 | 5123540 | 65713622 | 65713732 | SEQ ID NO: 804, SEQ ID NO: 805, SEQ ID NO: 806, SEQ ID NO: 807, SEQ ID NO: 808, SEQ ID NO: 809, SEQ ID NO: 810, SEQ ID NO: 811, SEQ ID NO: 812, SEQ ID NO: 813, SEQ ID NO: 814, SEQ ID NO: 815, SEQ ID NO: 816, SEQ ID NO: 817, SEQ ID NO: 818, SEQ ID NO: 819, SEQ ID NO: 820, SEQ ID NO: 821, SEQ ID NO: 822, SEQ ID NO: 823 |
| 17095583 | 5314892 | 91703547 | 91703806 | SEQ ID NO: 824, SEQ ID NO: 825, SEQ ID NO: 826, SEQ ID NO: 827, SEQ ID NO: 828, SEQ ID NO: 829, SEQ ID NO: 830, SEQ ID NO: 831, SEQ ID NO: 832, SEQ ID NO: 833, SEQ ID NO: 834, SEQ ID NO: 835, SEQ ID NO: 836, SEQ ID NO: 837, SEQ ID NO: 838, SEQ ID NO: 839, SEQ ID NO: 840, SEQ ID NO: 841 |
| 16757527 | 5052776 | 114845996 | 114846089 | SEQ ID NO: 842, SEQ ID NO: 843, SEQ ID NO: 844, SEQ ID NO: 845, SEQ ID NO: 846, SEQ ID NO: 847, SEQ ID NO: 848, SEQ ID NO: 849, SEQ ID NO: 850, SEQ ID NO: 851, SEQ ID NO: 852, SEQ ID NO: 853, SEQ ID NO: 854, SEQ ID NO: 855, SEQ ID NO: 856, SEQ ID NO: 857, SEQ ID NO: 858, SEQ ID NO: 859, SEQ ID NO: 860, SEQ ID NO: 861, SEQ ID NO: 862, SEQ ID NO: 863, SEQ ID NO: 864, SEQ ID NO: 865, SEQ ID NO: 866, SEQ ID NO: 867 |
| 17058221 | 5285817 | 65957267 | 65957293 | SEQ ID NO: 868, SEQ ID NO: 869, SEQ ID NO: 870, SEQ ID NO: 871, SEQ ID NO: 872, SEQ ID NO: 873, SEQ ID NO: 874, SEQ ID NO: 875, SEQ ID NO: 876, SEQ ID NO: 877, SEQ ID NO: 878, SEQ ID NO: 879 |
| 17025653 | 5262586 | 167271642 | 167271666 | SEQ ID NO: 880, SEQ ID NO: 881, SEQ ID NO: 882, SEQ ID NO: 883, SEQ ID NO: 884, SEQ ID NO: 885, SEQ ID NO: 886, SEQ ID NO: 887, SEQ ID NO: 888, SEQ ID NO: 889, SEQ ID NO: 890, SEQ ID NO: 891, SEQ ID NO: 892, SEQ ID NO: 893, SEQ ID NO: 894, SEQ ID NO: 895, SEQ ID NO: 896, SEQ ID NO: 897, SEQ ID NO: 898, SEQ ID NO: 899, SEQ ID NO: 900, SEQ ID NO: 901, SEQ ID NO: 902, SEQ ID NO: 903, SEQ ID NO: 904 |
| 16868954 | 5140844 | 11513296 | 11513320 | SEQ ID NO: 905, SEQ ID NO: 906, SEQ ID NO: 907, SEQ ID NO: 908, SEQ ID NO: 909, SEQ ID NO: 910, SEQ ID NO: 911, SEQ ID NO: 912, SEQ ID NO: 913, SEQ ID NO: 914, SEQ ID NO: 915, SEQ ID NO: 916, SEQ ID NO: 917, SEQ ID NO: 918, SEQ ID NO: 919, SEQ ID NO: 920 |
| 16877665 | 5147827 | 24248302 | 24248326 | SEQ ID NO: 921, SEQ ID NO: 922, SEQ ID NO: 923, SEQ ID NO: 924, SEQ ID NO: 925, SEQ ID NO: 926, SEQ ID NO: 927, SEQ ID NO: 928, SEQ ID NO: 929, SEQ ID NO: 930, SEQ ID NO: 931, SEQ ID NO: 932, SEQ ID NO: 933, SEQ ID NO: 934, SEQ ID NO: 935, SEQ ID NO: 936, SEQ ID NO: 937, SEQ ID NO: 938, SEQ ID NO: 939, SEQ ID NO: 940, SEQ ID NO: 941, SEQ ID NO: 942 |
| 17051908 | 5280941 | 134647642 | 134647666 | SEQ ID NO: 943, SEQ ID NO: 944, SEQ ID NO: 945, SEQ ID NO: 946, SEQ ID NO: 947, SEQ ID NO: 948, SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 964, SEQ ID NO: 965, SEQ ID NO: 966, SEQ ID NO: 967, SEQ ID NO: 968, SEQ ID NO: 969, SEQ ID NO: 970, SEQ ID NO: 971, SEQ ID NO: 972, SEQ ID NO: 973, SEQ ID NO: 974, SEQ ID NO: 975, SEQ ID NO: 976 |
| 17080053 | 5302714 | 106924048 | 106924072 | SEQ ID NO: 977, SEQ ID NO: 978, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, SEQ ID NO: 999, SEQ ID NO: 1,000, SEQ ID NO: 1,001, SEQ ID NO: 1,002 |
| 16842003 | 5118807 | 17875577 | 17875601 | SEQ ID NO: 1,003, SEQ ID NO: 1,004, SEQ ID NO: 1,005, SEQ ID NO: 1,006, SEQ ID NO: 1,007, SEQ ID NO: 1,008, SEQ ID NO: 1,009, SEQ ID NO: 1,010, SEQ ID NO: 1,011, SEQ ID NO: 1,012, SEQ ID NO: 1,013, SEQ ID NO: 1,014, SEQ ID NO: 1,015, SEQ ID NO: 1,016, SEQ ID NO: 1,017, SEQ ID NO: 1,018, SEQ ID NO: 1,019, SEQ ID NO: 1,020, SEQ ID NO: 1,021, SEQ ID NO: 1,022, SEQ ID NO: 1,023, SEQ ID NO: 1,024, SEQ ID NO: 1,025, SEQ ID NO: 1,026, SEQ ID NO: 1,027, SEQ ID NO: 1,028, SEQ ID NO: 1,029, SEQ ID NO: 1,030, SEQ ID NO: 1,031, SEQ ID NO: 1,032, SEQ ID NO: 1,033, SEQ ID NO: 1,034, SEQ ID NO: 1,035, SEQ ID NO: 1,036, SEQ ID NO: 1,037, SEQ ID NO: 1,038 |
| 17022799 | 5260293 | 112522836 | 112522860 | SEQ ID NO: 1,039, SEQ ID NO: 1,040, SEQ ID NO: 1,041, SEQ ID NO: 1,042, SEQ ID NO: 1,043, SEQ ID NO: 1,044, SEQ ID NO: 1,045, SEQ ID NO: 1,046, SEQ ID NO: 1,047, SEQ ID NO: 1,048, SEQ ID NO: 1,049, SEQ ID NO: 1,050, SEQ ID NO: 1,051, SEQ ID NO: 1,052, SEQ ID NO: 1,053, SEQ ID NO: 1,054, SEQ ID NO: 1,055, SEQ ID NO: 1,056, SEQ ID NO: 1,057, SEQ ID NO: 1,058, SEQ ID NO: 1,059, SEQ ID NO: 1,060, SEQ ID NO: 1,061, SEQ ID NO: 1,062, SEQ ID NO: 1,063, SEQ ID NO: 1,064, SEQ ID NO: 1,065, SEQ ID NO: 1,066, SEQ ID NO: 1,067, SEQ ID NO: 1,068, SEQ ID NO: 1,069, SEQ ID NO: 1,070, SEQ ID NO: 1,071, SEQ ID NO: 1,072, SEQ ID NO: 1,073, SEQ ID NO: 1,074, SEQ ID NO: 1,075, SEQ ID NO: 1,076, SEQ ID NO: 1,077, SEQ ID NO: 1,078, SEQ ID NO: 1,079, SEQ ID NO: 1,080, SEQ ID NO: 1,081, SEQ ID NO: 1,082, SEQ ID NO: 1,083, SEQ ID NO: 1,084, SEQ ID NO: 1,085, SEQ ID NO: 1,086, SEQ ID NO: 1,087, SEQ ID NO: 1,088, SEQ ID NO: 1,089, SEQ ID NO: 1,090, SEQ ID NO: 1,091, SEQ ID NO: 1,092, SEQ ID NO: 1,093, SEQ ID NO: 1,094, SEQ ID NO: 1,095, SEQ ID NO: 1,096, SEQ ID NO: 1,097, SEQ ID NO: 1,098, SEQ ID NO: 1,099, SEQ ID NO: 1,100, SEQ ID NO: 1,101 |
| 16786369 | 5074755 | 74416815 | 74416839 | SEQ ID NO: 1,102, SEQ ID NO: 1,103, SEQ ID NO: 1,104, SEQ ID NO: 1,105, SEQ ID NO: 1,106, SEQ ID NO: 1,107, SEQ ID NO: 1,108, SEQ ID NO: 1,109, SEQ ID NO: 1,110, SEQ ID NO: 1,111, SEQ ID NO: 1,112, SEQ ID NO: 1,113, SEQ ID NO: 1,114, SEQ ID NO: 1,115, SEQ ID NO: 1,116, SEQ ID NO: 1,117, SEQ ID NO: 1,118, SEQ ID NO: 1,119, SEQ ID NO: 1,120, SEQ ID NO: 1,121, SEQ ID NO: 1,122, SEQ ID NO: 1,123, SEQ ID NO: 1,124, SEQ ID NO: 1,125, SEQ ID NO: 1,126, SEQ ID NO: 1,127, SEQ ID NO: 1,128, SEQ ID NO: 1,129, SEQ ID NO: 1,130 |
| 16920377 | 5181208 | 50409490 | 50409566 | SEQ ID NO: 1,131, SEQ ID NO: 1,132, SEQ ID NO: 1,133, SEQ ID NO: 1,134, SEQ ID NO: 1,135, SEQ ID NO: 1,136, SEQ ID NO: 1,137, SEQ ID NO: 1,138, SEQ ID NO: 1,139, SEQ ID NO: 1,140, SEQ ID NO: 1,141, SEQ ID NO: 1,142, SEQ ID NO: 1,143, SEQ ID NO: 1,144, SEQ ID NO: 1,145, SEQ ID NO: 1,146, SEQ ID NO: 1,147, SEQ ID NO: 1,148, SEQ ID NO: 1,149, SEQ ID NO: 1,150, SEQ ID NO: 1,151, SEQ ID NO: 1,152, SEQ ID NO: 1,153, SEQ ID NO: 1,154, SEQ ID NO: 1,155, SEQ ID NO: 1,156, SEQ ID NO: 1,157, SEQ ID NO: 1,158 |
| 16851430 | 5126344 | 20735797 | 20735851 | SEQ ID NO: 1,159, SEQ ID NO: 1,160, SEQ ID NO: 1,161, SEQ ID NO: 1,162, SEQ ID NO: 1,163, SEQ ID NO: 1,164, SEQ ID NO: 1,165, SEQ ID NO: 1,166, SEQ ID NO: 1,167, SEQ ID NO: 1,168, SEQ ID NO: 1,169, SEQ ID NO: 1,170, SEQ ID NO: 1,171, SEQ ID NO: 1,172, SEQ ID NO: 1,173, SEQ ID NO: 1,174, SEQ ID NO: 1,175, SEQ ID NO: 1,176, SEQ ID NO: 1,177, SEQ ID NO: 1,178, SEQ ID NO: 1,179, SEQ ID NO: 1,180, SEQ ID NO: 1,181, SEQ ID NO: 1,182, SEQ ID NO: 1,183 |
| 16866470 | 5138794 | 622162 | 622229 | SEQ ID NO: 1,184, SEQ ID NO: 1,185, SEQ ID NO: 1,186, SEQ ID NO: 1,187, SEQ ID NO: 1,188, SEQ ID NO: 1,189, SEQ ID NO: 1,190, SEQ ID NO: 1,191, SEQ ID NO: 1,192, SEQ ID NO: 1,193, SEQ ID NO: 1,194, SEQ ID NO: 1,195, SEQ ID NO: 1,196, SEQ ID NO: 1,197, SEQ ID NO: 1,198, SEQ ID NO: 1,199, SEQ ID NO: 1,200, SEQ ID NO: 1,201, SEQ ID NO: 1,202, SEQ ID NO: 1,203, SEQ ID NO: 1,204, SEQ ID NO: 1,205, SEQ ID NO: 1,206, SEQ ID NO: 1,207, SEQ ID NO: 1,208, SEQ ID NO: 1,209, SEQ ID NO: 1,210 |
| 17000583 | 5243330 | 138716329 | 138716372 | SEQ ID NO: 1,211, SEQ ID NO: 1,212, SEQ ID NO: 1,213, SEQ ID NO: 1,214, SEQ ID NO: 1,215, SEQ ID NO: 1,216, SEQ ID NO: 1,217, SEQ ID NO: 1,218, SEQ ID NO: 1,219, SEQ ID NO: 1,220, SEQ ID NO: 1,221, SEQ ID NO: 1,222, SEQ ID NO: 1,223, SEQ ID NO: 1,224, SEQ ID NO: 1,225, SEQ ID NO: 1,226, SEQ ID NO: 1,227, SEQ ID NO: 1,228, SEQ ID NO: 1,229, SEQ ID NO: 1,230, SEQ ID NO: 1,231, SEQ ID NO: 1,232, SEQ ID NO: 1,233, SEQ ID NO: 1,234, SEQ ID NO: 1,235, SEQ ID NO: 1,236, SEQ ID NO: 1,237, SEQ ID NO: 1,238, SEQ ID NO: 1,239, SEQ ID NO: 1,240 |
| 17019044 | 5257313 | 41162239 | 41162438 | SEQ ID NO: 1,241, SEQ ID NO: 1,242, SEQ ID NO: 1,243, SEQ ID NO: 1,244, SEQ ID NO: 1,245, SEQ ID NO: 1,246, SEQ ID NO: 1,247, SEQ ID NO: 1,248, SEQ ID NO: 1,249, SEQ ID NO: 1,250, SEQ ID NO: 1,251, SEQ ID NO: 1,252, SEQ ID NO: 1,253, SEQ ID NO: 1,254, SEQ ID NO: 1,255, SEQ ID NO: 1,256 |
| 17057414 | 5285139 | 45022748 | 45022834 | SEQ ID NO: 1,257, SEQ ID NO: 1,258, SEQ ID NO: 1,259, SEQ ID NO: 1,260, SEQ ID NO: 1,261, SEQ ID NO: 1,262, SEQ ID NO: 1,263, SEQ ID NO: 1,264, SEQ ID NO: 1,265, SEQ ID NO: 1,266, SEQ ID NO: 1,267, SEQ ID NO: 1,268, SEQ ID NO: 1,269, SEQ ID NO: 1,270, SEQ ID NO: 1,271, SEQ ID NO: 1,272, SEQ ID NO: 1,273, SEQ ID NO: 1,274, SEQ ID NO: 1,275, SEQ ID NO: 1,276 |
| 17059640 | 5286964 | 91758297 | 91758322 | SEQ ID NO: 1,277, SEQ ID NO: 1,278, SEQ ID NO: 1,279, SEQ ID NO: 1,280, SEQ ID NO: 1,281, SEQ ID NO: 1,282, SEQ ID NO: 1,283, SEQ ID NO: 1,284, SEQ ID NO: 1,285, SEQ ID NO: 1,286, SEQ ID NO: 1,287, SEQ ID NO: 1,288, SEQ ID NO: 1,289, SEQ ID NO: 1,290, SEQ ID NO: 1,291, SEQ ID NO: 1,292, SEQ ID NO: 1,293, SEQ ID NO: 1,294, SEQ ID NO: 1,295, SEQ ID NO: 1,296, SEQ ID NO: 1,297, SEQ ID NO: 1,298, SEQ ID NO: 1,299 |
| 17076434 | 5299845 | 38280311 | 38280335 | SEQ ID NO: 1,300, SEQ ID NO: 1,301, SEQ ID NO: 1,302, SEQ ID NO: 1,303, SEQ ID NO: 1,304, SEQ ID NO: 1,305, SEQ ID NO: 1,306, SEQ ID NO: 1,307, SEQ ID NO: 1,308, SEQ ID NO: 1,309, SEQ ID NO: 1,310, SEQ ID NO: 1,311, SEQ ID NO: 1,312, SEQ ID NO: 1,313, SEQ ID NO: 1,314, SEQ ID NO: 1,315, SEQ ID NO: 1,316, SEQ ID NO: 1,317, SEQ ID NO: 1,318, SEQ ID NO: 1,319, SEQ ID NO: 1,320, SEQ ID NO: 1,321, SEQ ID NO: 1,322, SEQ ID NO: 1,323, SEQ ID NO: 1,324, SEQ ID NO: 1,325, SEQ ID NO: 1,326, SEQ ID NO: 1,327, SEQ ID NO: 1,328, SEQ ID NO: 1,329, SEQ ID NO: 1,330, SEQ ID NO: 1,331, SEQ ID NO: 1,332, SEQ ID NO: 1,333, SEQ ID NO: 1,334, SEQ ID NO: 1,335 |
| 17114699 | 5329708 | 139805916 | 139805949 | SEQ ID NO: 1,336, SEQ ID NO: 1,337, SEQ ID NO: 1,338, SEQ ID NO: 1,339, SEQ ID NO: 1,340, SEQ ID NO: 1,341, SEQ ID NO: 1,342, SEQ ID NO: 1,343, SEQ ID NO: 1,344, SEQ ID NO: 1,345, SEQ ID NO: 1,346, SEQ ID NO: 1,347, SEQ ID NO: 1,348 |

**Table 6. Optimal Set of 16 probe sets that identifies specific exons in the RNA biomarker, which are used for detecting that expression is decreased or increased of RNA biomarkers in a sample comprising total polynucleotides isolated from CD45-positive cells (or biofluids) from a male subject, corresponding Probe Set IDs the Exon_ID, the start and end of the exon and the nucleic acid sequences of the probes (Nucleic Acid Sequences of Probes ) within each probe set for the male subjects.**

| **PROBE SET ID** | **EXON_ID** | **START** | **STOP** | **NUCLEIC ACID SEQUENCES OF PROBES** |
|---|---|---|---|---|
| 17059640 | 5286964 | 91758297 | 91758322 | SEQ ID NO: 1,277, SEQ ID NO: 1,278, SEQ ID NO: 1,279, SEQ ID NO: 1,280, SEQ ID NO: 1,281, SEQ ID NO: 1,282, SEQ ID NO: 1,283, SEQ ID NO: 1,284, SEQ ID NO: 1,285, SEQ ID NO: 1,286, SEQ ID NO: 1,287, SEQ ID NO: 1,288, SEQ ID NO: 1,289, SEQ ID NO: 1,290, SEQ ID NO: 1,291, SEQ ID NO: 1,292, SEQ ID NO: 1,293, SEQ ID NO: 1,294, SEQ ID NO: 1,295, SEQ ID NO: 1,296, SEQ ID NO: 1,297, SEQ ID NO: 1,298, SEQ ID NO: 1,299 |
| 17019044 | 5257313 | 41162239 | 41162438 | SEQ ID NO: 1,241, SEQ ID NO: 1,242, SEQ ID NO: 1,243, SEQ ID NO: 1,244, SEQ ID NO: 1,245, SEQ ID NO: 1,246, SEQ ID NO: 1,247, SEQ ID NO: 1,248, SEQ ID NO: 1,249, SEQ ID NO: 1,250, SEQ ID NO: 1,251, SEQ ID NO: 1,252, SEQ ID NO: 1,253, SEQ ID NO: 1,254, SEQ ID NO: 1,255, SEQ ID NO: 1,256 |
| 17076434 | 5299845 | 38280311 | 38280335 | SEQ ID NO: 1,300, SEQ ID NO: 1,301, SEQ ID NO: 1,302, SEQ ID NO: 1,303, SEQ ID NO: 1,304, SEQ ID NO: 1,305, SEQ ID NO: 1,306, SEQ ID NO: 1,307, SEQ ID NO: 1,308, SEQ ID NO: 1,309, SEQ ID NO: 1,310, SEQ ID NO: 1,311, SEQ ID NO: 1,312, SEQ ID NO: 1,313, SEQ ID NO: 1,314, SEQ ID NO: 1,315, SEQ ID NO: 1,316, SEQ ID NO: 1,317, SEQ ID NO: 1,318, SEQ ID NO: 1,319, SEQ ID NO: 1,320, SEQ ID NO: 1,321, SEQ ID NO: 1,322, SEQ ID NO: 1,323, SEQ ID NO: 1,324, SEQ ID NO: 1,325, SEQ ID NO: 1,326, SEQ ID NO: 1,327, SEQ ID NO: 1,328, SEQ ID NO: 1,329, SEQ ID NO: 1,330, SEQ ID NO: 1,331, SEQ ID NO: 1,332, SEQ ID NO: 1,333, SEQ ID NO: 1,334, SEQ ID NO: 1,335 |
| 16945454 | 5200110 | 129695566 | 129696022 | SEQ ID NO: 782, SEQ ID NO: 783, SEQ ID NO: 784, SEQ ID NO: 785, SEQ ID NO: 786, SEQ ID NO: 787, SEQ ID NO: 788, SEQ ID NO: 789, SEQ ID NO: 790, SEQ ID NO: 791, SEQ ID NO: 792, SEQ ID NO: 793, SEQ ID NO: 794, SEQ ID NO: 795, SEQ ID NO: 796, SEQ ID NO: 797, SEQ ID NO: 798, SEQ ID NO: 799, SEQ ID NO: 800, SEQ ID NO: 801, SEQ ID NO: 802, SEQ ID NO: 803 |
| 17058221 | 5285817 | 65957267 | 65957293 | SEQ ID NO: 868, SEQ ID NO: 869, SEQ ID NO: 870, SEQ ID NO: 871, SEQ ID NO: 872, SEQ ID NO: 873, SEQ ID NO: 874, SEQ ID NO: 875, SEQ ID NO: 876, SEQ ID NO: 877, SEQ ID NO: 878, SEQ ID NO: 879 |
| 17000583 | 5243330 | 138716329 | 138716372 | SEQ ID NO: 1,211, SEQ ID NO: 1,212, SEQ ID NO: 1,213, SEQ ID NO: 1,214, SEQ ID NO: 1,215, SEQ ID NO: 1,216, SEQ ID NO: 1,217, SEQ ID NO: 1,218, SEQ ID NO: 1,219, SEQ ID NO: 1,220, SEQ ID NO: 1,221, SEQ ID NO: 1,222, SEQ ID NO: 1,223, SEQ ID NO: 1,224, SEQ ID NO: 1,225, SEQ ID NO: 1,226, SEQ ID NO: 1,227, SEQ ID NO: 1,228, SEQ ID NO: 1,229, SEQ ID NO: 1,230, SEQ ID NO: 1,231, SEQ ID NO: 1,232, SEQ ID NO: 1,233, SEQ ID NO: 1,234, SEQ ID NO: 1,235, SEQ ID NO: 1,236, SEQ ID NO: 1,237, SEQ ID NO: 1,238, SEQ ID NO: 1,239, SEQ ID NO: 1,240 |
| 17114699 | 5329708 | 139805916 | 139805949 | SEQ ID NO: 1,336, SEQ ID NO: 1,337, SEQ ID NO: 1,338, SEQ ID NO: 1,339, SEQ ID NO: 1,340, SEQ ID NO: 1,341, SEQ ID NO: 1,342, SEQ ID NO: 1,343, SEQ ID NO: 1,344, SEQ ID NO: 1,345, SEQ ID NO: 1,346, SEQ ID NO: 1,347, SEQ ID NO: 1,348 |
| 16866470 | 5138794 | 622162 | 622229 | SEQ ID NO: 1,184, SEQ ID NO: 1,185, SEQ ID NO: 1,186, SEQ ID NO: 1,187, SEQ ID NO: 1,188, SEQ ID NO: 1,189, SEQ ID NO: 1,190, SEQ ID NO: 1,191, SEQ ID NO: 1,192, SEQ ID NO: 1,193, SEQ ID NO: 1,194, SEQ ID NO: 1,195, SEQ ID NO: 1,196, SEQ ID NO: 1,197, SEQ ID NO: 1,198, SEQ ID NO: 1,199, SEQ ID NO: 1,200, SEQ ID NO: 1,201, SEQ ID NO: 1,202, SEQ ID NO: 1,203, SEQ ID NO: 1,204, SEQ ID NO: 1,205, SEQ ID NO: 1,206, SEQ ID NO: 1,207, SEQ ID NO: 1,208, SEQ ID NO: 1,209, SEQ ID NO: 1,210 |
| 17080053 | 5302714 | 106924048 | 106924072 | SEQ ID NO: 977, SEQ ID NO: 978, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, SEQ ID NO: 999, SEQ ID NO: 1,000, SEQ ID NO: 1,001, SEQ ID NO: 1,002 |
| 16786369 | 5074755 | 74416815 | 74416839 | SEQ ID NO: 1,102, SEQ ID NO: 1,103, SEQ ID NO: 1,104, SEQ ID NO: 1,105, SEQ ID NO: 1,106, SEQ ID NO: 1,107, SEQ ID NO: 1,108, SEQ ID NO: 1,109, SEQ ID NO: 1,110, SEQ ID NO: 1,111, SEQ ID NO: 1,112, SEQ ID NO: 1,113, SEQ ID NO: 1,114, SEQ ID NO: 1,115, SEQ ID NO: 1,116, SEQ ID NO: 1,117, SEQ ID NO: 1,118, SEQ ID NO: 1,119, SEQ ID NO: 1,120, SEQ ID NO: 1,121, SEQ ID NO: 1,122, SEQ ID NO: 1,123, SEQ ID NO: 1,124, SEQ ID NO: 1,125, SEQ ID NO: 1,126, SEQ ID NO: 1,127, SEQ ID NO: 1,128, SEQ ID NO: 1,129, SEQ ID NO: 1,130 |
| 17118935 | 0 | 17 | 47 | SEQ ID NO: 775, SEQ ID NO: 776, SEQ ID NO: 777, SEQ ID NO: 778, SEQ ID NO: 779, SEQ ID NO: 780, SEQ ID NO: 781 |
| 16877665 | 5147827 | 24248302 | 24248326 | SEQ ID NO: 921, SEQ ID NO: 922, SEQ ID NO: 923, SEQ ID NO: 924, SEQ ID NO: 925, SEQ ID NO: 926, SEQ ID NO: 927, SEQ ID NO: 928, SEQ ID NO: 929, SEQ ID NO: 930, SEQ ID NO: 931, SEQ ID NO: 932, SEQ ID NO: 933, SEQ ID NO: 934, SEQ ID NO: 935, SEQ ID NO: 936, SEQ ID NO: 937, SEQ ID NO: 938, SEQ ID NO: 939, SEQ ID NO: 940, SEQ ID NO: 941, SEQ ID NO: 942 |
| 17057414 | 5285139 | 45022748 | 45022834 | SEQ ID NO: 1,257, SEQ ID NO: 1,258, SEQ ID NO: 1,259, SEQ ID NO: 1,260, SEQ ID NO: 1,261, SEQ ID NO: 1,262, SEQ ID NO: 1,263, SEQ ID NO: 1,264, SEQ ID NO: 1,265, SEQ ID NO: 1,266, SEQ ID NO: 1,267, SEQ ID NO: 1,268, SEQ ID NO: 1,269, SEQ ID NO: 1,270, SEQ ID NO: 1,271, SEQ ID NO: 1,272, SEQ ID NO: 1,273, SEQ ID NO: 1,274, SEQ ID NO: 1,275, SEQ ID NO: 1,276 |
| 16851430 | 5126344 | 20735797 | 20735851 | SEQ ID NO: 1,159, SEQ ID NO: 1,160, SEQ ID NO: 1,161, SEQ ID NO: 1,162, SEQ ID NO: 1,163, SEQ ID NO: 1,164, SEQ ID NO: 1,165, SEQ ID NO: 1,166, SEQ ID NO: 1,167, SEQ ID NO: 1,168, SEQ ID NO: 1,169, SEQ ID NO: 1,170, SEQ ID NO: 1,171, SEQ ID NO: 1,172, SEQ ID NO: 1,173, SEQ ID NO: 1,174, SEQ ID NO: 1,175, SEQ ID NO: 1,176, SEQ ID NO: 1,177, SEQ ID NO: 1,178, SEQ ID NO: 1,179, SEQ ID NO: 1,180, SEQ ID NO: 1,181, SEQ ID NO: 1,182, SEQ ID NO: 1,183 |
| 16868954 | 5140844 | 11513296 | 11513320 | SEQ ID NO: 905, SEQ ID NO: 906, SEQ ID NO: 907, SEQ ID NO: 908, SEQ ID NO: 909, SEQ ID NO: 910, SEQ ID NO: 911, SEQ ID NO: 912, SEQ ID NO: 913, SEQ ID NO: 914, SEQ ID NO: 915, SEQ ID NO: 916, SEQ ID NO: 917, SEQ ID NO: 918, SEQ ID NO: 919, SEQ ID NO: 920 |
| 16831561 | 5110382 | 16926501 | 16926546 | SEQ ID NO: 704, SEQ ID NO: 705, SEQ ID NO: 706, SEQ ID NO: 707, SEQ ID NO: 708, SEQ ID NO: 709, SEQ ID NO: 710, SEQ ID NO: 711, SEQ ID NO: 712, SEQ ID NO: 713, SEQ ID NO: 714, SEQ ID NO: 715, SEQ ID NO: 716, SEQ ID NO: 717, SEQ ID NO: 718, SEQ ID NO: 719, SEQ ID NO: 720, SEQ ID NO: 721, SEQ ID NO: 722 |

In a preferred embodiment, CD45+ MNC cells may be isolated from a blood sample or tissue or biofluids sample taken from a female or male subject using conventional methods, for instance CD45+ MNC cells may be isolated from the blood sample using conventional blood cell isolation techniques and immunohistochemistry techniques (e.g. using antibodies which are specific for capturing or binding CD45+ MNC cells, such as CD32 Monoclonal Antibodies by Invitrogen (6C4 (CD32)), Functional Grade, eBioscience). Alternatively, the specific RNA biomarker panel may be detected in unsorted cells from blood samples (as long as (1) CD45+ MNC cells are present, or (2) have been present, or (3) have been able to secrete the RNA biomarker into the biofluids). The skilled person is well-acquainted with CD45+ MNC cells and is aware of methods for isolating them from the blood. In a subsequent step, total polynucleotides may be isolated from said CD45+ MNC cells or biofluids, which in turn may be used for detecting the expression levels of the RNA biomarkers of the invention, using the probes as taught herein (or conventional antibody detection methods).

In the methods of the invention (i.e. for both female-specific and male-specific methods), the term 'making a diagnosis' or 'diagnosing' as used herein refers to the act of determining which disease(s) or condition(s) explains a person's symptoms and signs. The information required for diagnosis is typically collected from a history and physical examination of the person (female or male) seeking medical care. Often, two or more diagnostic procedures, such as diagnostic tests, are also done during the process. Diagnostic tests allow physicians to make informed decisions about treatment and establish a prognosis. The act of 'make a diagnosis' or 'diagnosing' (as well as the opinion reached thereby) does not necessarily involve elucidation of the aetiology of the diseases or conditions of interest. Additionally, for follow up / monitoring of patients (the natural progression of disease).

In an embodiment the IVDC is selected from the group consisting of myocardial ischemia, myocardial infarction and angina pectoris. In a preferred embodiment, the IVDC is myocardial ischemia, preferably early onset myocardial ischemia/ silent ischemia, as discussed above.

In a preferred embodiment the method of the invention concerns an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level, d. determining a second expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a further test sample collected from said subject at a later time point than the first test sample, e. comparing the first expression level of each RNA biomarker with the second expression level of each RNA biomarker, f. diagnosing whether the progression of IVD and/or IVDC over time in said female subject has occurred based on the difference of at least two RNA biomarkers from said group between the first expression level and the second expression level in said female subject.

In a preferred embodiment the method of the invention concerns an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level, d. administering a treatment for IVD and/or IVDC after step c., e. determining a second expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a further test sample collected from said subject at a later time point than the first test sample, f. comparing the first expression level of each RNA biomarker with the second expression level of each RNA biomarker, g. determining whether the treatment for IVD and/or IVDC has improved the occurrence and/or severity of IVD and/or IVDC in said female subject based on the difference of at least two RNA biomarkers from said group between the first expression level and the second expression level in said female subject.

In a preferred embodiment the method of the invention concerns an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least five, preferably at least ten RNA biomarkers from said group are different between the first expression level and the control expression level.

In a preferred embodiment the method of the invention concerns an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least fourteen RNA biomarkers from said group are different between the first expression level and the control expression level.

In a preferred embodiment the method of the invention concerns an *in vitro* method for diagnosis of occurrence and/or assessing severity of ischemic vascular disease (IVD) and/or ischemic vascular disease-related complication (IVDC) in a female subject, said method comprising the steps of a. determining a first expression level of each RNA biomarker from the group comprising ADAMTS8, AK094845, AMOT, BTRC, CNTNAP3, COL2A1, DCHS2, DENND5B, LPAR6, MMP11, NBLA00301, PRSS33, QTRTD1 and TDG, in CD45+ mononuclear cells (MNC) from a test sample collected from said female subject, and b. comparing said first expression level of each RNA biomarker from the group comprising ADAMTS8, AK094845, AMOT, BTRC, CNTNAP3, COL2A1, DCHS2, DENND5B, LPAR6, MMP11, NBLA00301, PRSS33, QTRTD1 and TDG, in CD45+ mononuclear cells (MNC) collected from said female subject with a control expression level of each RNA biomarker from the group comprising ADAMTS8, AK094845, AMOT, BTRC, CNTNAP3, COL2A1, DCHS2, DENND5B, LPAR6, MMP11, NBLA00301, PRSS33, QTRTD1 and TDG from a control sample collected from control female subject(s), c. diagnosing occurrence and/or assessing severity of IVD and/or IVDC based on the differences between the first expression level and the control expression level, wherein it is determined that IVD and/or IVDC occurred or is severe if at least fourteen RNA biomarkers from said group are different between the first expression level and the control expression level.

In a preferred embodiment the method of the invention concerns an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level, d. determining a second expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a further test sample collected from said subject at a later time point than the first test sample, e. comparing the first expression level of each RNA biomarker with the second expression level of each RNA biomarker, f. diagnosing whether the progression of IVD and/or IVDC over time in said male subject has occurred based on the difference of at least two RNA biomarkers from said group between the first expression level and the second expression level in said male subject.

In a preferred embodiment the method of the invention concerns an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least two RNA biomarkers from said group are different between the first expression level and the control expression level, d. administering a treatment for IVD and/or IVDC after step c., e. determining a second expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a further test sample collected from said subject at a later time point than the first test sample, g. comparing the first expression level of each RNA biomarker with the second expression level of each RNA biomarker, h. determining whether the treatment for IVD and/or IVDC has improved the occurrence and/or severity of IVD and/or IVDC in said male subject based on the difference of at least two RNA biomarkers from said group between the first expression level and the second expression level in said male subject.

In a preferred embodiment the method of the invention concerns an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at least ten, preferably at least fourteen, RNA biomarkers from said group are different between the first expression level and the control expression level.

In a preferred embodiment the method of the invention concerns an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s), c. diagnosing occurrence and/or assessing severity of IVD and/or IVDC based on the differences between the first expression level and the control expression level, wherein it is determined that IVD and/or IVDC occurred or is severe at least sixteen, preferably at least eighteen RNA biomarkers from said group are different between the first expression level and the control expression level.

In a preferred embodiment the method of the invention concerns an *in vitro* method for diagnosis of occurrence and/or assessing severity of ischemic vascular disease (IVD) and/or ischemic vascular disease-related complication (IVDC) in a male subject, said method comprising the steps of a. determining a first expression level of each RNA biomarker from the group comprising CYP51A1 , TREML2, ProbSetID_17118935, TRH, TCONS_I2_00026656-XLOC_I2_013873, SLC23A1, TCONS_00017554-XLOC_008249, POLRMT , ENST00000518932, COQ6, ExonID_5299845, MFSD2B, SNHG15, CABLES1, RGL3 and TCONS_00025321-XLOC_012127, in CD45+ mononuclear cells (MNC) from a test sample collected from said male subject, and b. comparing said first expression level of each RNA biomarker from the group comprising CYP51A1 , TREML2, ProbSetID_17118935, TRH, TCONS_I2_00026656-XLOC_I2_013873, SLC23A1, TCONS_00017554-XLOC_008249, POLRMT , ENST00000518932, COQ6, ExonID_5299845, MFSD2B, SNHG15, CABLES1, RGL3 and TCONS_00025321-XLOC_012127, in CD45+ mononuclear cells (MNC) collected from said male subject with a control expression level of each RNA biomarker from the group comprising CYP51A1 , TREML2, ProbSetID_17118935, TRH, TCONS_I2_00026656-XLOC_I2_013873, SLC23A1, TCONS_00017554-XLOC_008249, POLRMT , ENST00000518932, COQ6, ExonID_5299845, MFSD2B, SNHG15, CABLES1, RGL3 and TCONS_00025321-XLOC_012127 from a control sample collected from control male subject(s), c. diagnosing occurrence and/or assessing severity of IVD and/or IVDC based on the differences between the first expression level and the control expression level, wherein it is determined that IVD and/or IVDC occurred or is severe if at sixteen RNA biomarkers from said group are different between the first expression level and the control expression level.

### FIGURE DESCRIPTION

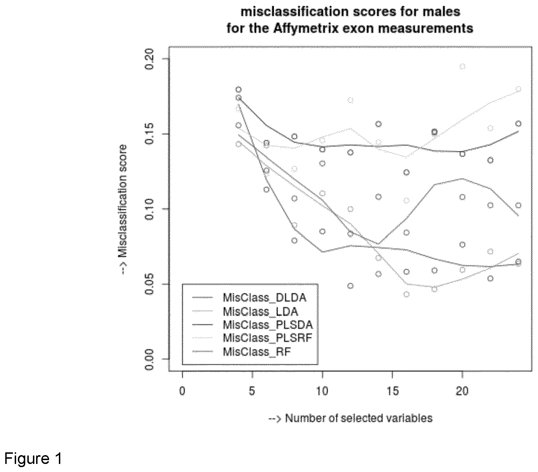

Figure 1: Misclassification scores for the classification models depending on the number of optimized male exons (Number of selected variables) of identified RNA biomarkers and prediction models/methods used (DLDA, FDA, LDA, PLSDA, PLSRF, QDA and RF). The Linear Discriminant Analysis (LDA) gave an optimal RNA biomarker set of 16 RNA biomarkers.

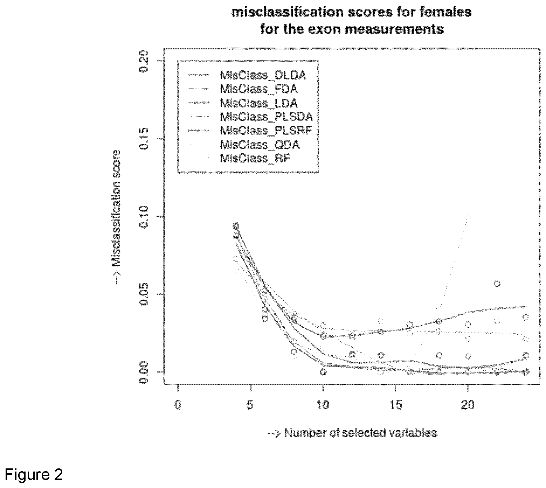

Figure 2: Misclassification scores for the classification models depending on the number of optimized female exons (Number of selected variables) of identified RNA biomarkers and prediction models/methods used (DLDA, FDA, LDA, PLSDA, PLSRF, QDA and RF). The Linear Discriminant Analysis (LDA) gave an optimal RNA biomarker set of 14 RNA biomarkers.

### EXAMPLES

### Example 1:

### Female-specific study

A study was performed to assess the pattern of expression of the total polynucleotides expressed in or on CD45+ MNC cells obtained from female patients (n=31) suffering from myocardial ischemia vs female control subjects in which myocardial ischemia was excluded (n=91). the goal of the study was, to first, determine whether any differential pattern of expression of the total polynucleotides expressed in or on CD45+ MNC cells exist between female patients suffering from myocardial ischemia as compared to control subjects, and second, to use the results (i.e. the differentially expressed polynucleotides) to identify which differentially expressed polynucleotides may be used as a RNA biomarker for diagnosing, detecting the occurrence of, progression of, quantifying myocardial ischemia and/or monitoring myocardial ischemia over time or in response to treatment in female subject or patient.

Patients suffering from myocardial ischemia and control subjects were selected as follows. Female subjects were referred by the primary physician to be examined for chest pain complaints (e.g. myocardial ischemia or angina pectoris) at the cardiology outpatient clinic. Chest pain and/or chest discomfort may be caused by myocardial ischemia, but may also be caused by unrelated diseases, including anxiety, gastrointestinal complaints (for instance gastric reflux, stomach ulcer), or muscle ache. Therefore, to rule out unrelated conditions and to select patients suffering from myocardial ischemia, patients were screened using standard protocols for anamnesis, ECG, blood analysis, exercise testing (ergometry) and multi-slice CT scan. In case of ambiguous results, the patients were subjected to nuclear myocardial perfusion imaging (MIBI SPECT analysis) to rule out any myocardial ischemia. Patients suffering from myocardial ischemia, which were selected based on the battery of tests described therein, provided blood samples for further analysis (e.g. isolation of total polynucleotides from immune-isolated CD45+ cells and subsequent polynucleotide expression profiling using a commercial Affymetrix Human Gene 2.1 ST array). Blood samples were also obtained from healthy subjects that also have been referred for further diagnosis of chest discomfort, however myocardial ischemia was ruled out using standard of care diagnosis (using ergometry, MSCT and MIBI SPECT). These patients also received a telephone follow-up at 3 months after this diagnosis to ensure the absence of any cardiac event during the 3 months following the diagnosis (including any admission or intervention due to angina pectoris). The samples from the healthy subjects served as a control group.

### Example 2

### Male-specific study

The experimental procedure of example 1 was repeated for male patients (n=98) suffering from myocardial ischemia and male control subjects not suffering from myocardial ischemia (n=84).

### Example 3

### Isolation of CD45+ MNC cells from blood samples

The protocol below was applied to blood samples obtained from all patients (both sexes, with and without manifest myocardial ischemia).

The following buffers and mediums were prepared according to standard procedures:
- Buffer 1: consists of a PBS buffer with 0,4% EDTA.
- Buffer 2: consists of a PBS buffer with 0,2% EDTA and 2% FCS.
- Freeze medium: consists of a DMEM medium with 10% FCS and 10% DMSO.

The blood samples were processed as follows:
- step 1: A total of 6 tubes of whole blood for each patient were collected and labelled accordingly.
- step 2: an amount of 20 ml of whole blood was transferred in one 50 cc tube and 28 ml of with buffer was added.
- step 3: A total of 12 tubes containing 9 ml of Ficoll (used to separate blood to its components) were prepared per patient.
- step 4: An amount of 12 ml of dilated blood (obtained from step 2) was added to each tube of step 3.
- step 5: The tubes of step 4 were centrifuged at a speed of 1400 rpm, for 40 minutes.
- step 6: The buffy coat present at the surface of the tubes of step 5 was gently pipetted and transferred to a new tube. Specifically, an amount corresponding to the buffy coats taken from 4 tubes (step 5) was transferred into one tube, after which the volume in the tube was completed to 50 ml with buffer 2.
- step 7: The tubes of step 6 were centrifuged at a speed of 1400 rpm, for 10min.
- step 8: The pellet (containing the CD45+ MNC cells) formed in each tube (step 7) was re-dissolved in 10 ml of buffer 2.
- step 9: An amount of 5 ml of CD45+ cell solution (from step 8) was pipetted into 2 new tubes.
- step 10: The tubes of step 9 were centrifuged at a speed of 1400 rpm, for 10min.
- step 11: The supernatant (from the tube of step 10) was decanted and allowed to dry.
- step 12: The pellet formed in the tubes of step 11, was put in a new tube (1 pellet/tube) on ice and re-dissolved in 3 ml of freeze medium and stored at -80 °C.
- step 13: After approximately 1 week, the samples of step 12 were thawed and dissolved in buffer 2 (1 pellet in 100 ul buffer 2).
- step 14: the CD45+ MNC cells were recovered using a CD32 antibody according to standard immunohistochemistry procedures.

### Example 4:

Detecting the expression level of total polynucleotides isolated from CD45-positive cells The protocol below was applied to both blood samples obtained from female and male patients suffering from myocardial ischemia and female and male control subjects not suffering from myocardial ischemia

Total RNA was isolated from CD45+ MNC cells, which themselves were isolated from blood sample from subjects (i.e. female or male suffering from myocardial ischemia) and control subjects (i.e. female or male subjects not suffering from myocardial ischemia) according to standard protocols.

A genome wide polynucleotide expression exon microarray analysis was performed according to the manufacturer protocol (Affymetrix Human Gene 2.1 ST Exon Microarray, Affymetrix Santa Clara, CA, USA) on the total RNA that was isolated from CD45+ MNC cells obtained from each experimental group and for each sex so as to identify potential RNA biomarkers that are differentially expressed in CD45+ circulating cells derived from sample obtained from female and male patients suffering from myocardial ischemia (i.e. angina pectoris) vs. control female and male subjects in which myocardial ischemia was ruled out. The polynucleotide expression exon microarray analysis was performed according to the manufacturer's instructions. The relative polynucleotide expression profile between female patients suffering from myocardial ischemia and female control subjects was established. The same was performed for the male patients suffering from myocardial ischemia and male control subjects. An in depth bioinformatical analysis was performed to determine the probe sets (thereby RNA biomarkers) being indicative of IVD or IVDC in respectively female and male subjects (see Example 5).

### Example 5:

### Statistical Analysis of Affymetrix Output for Female and Male Specific RNA biomarkers and Number of RNA biomarkers

Affymetrix software was used to extract, normalize, and summarize intensity data from the Affymetrix Exon Microarray Human Gene 2.1 ST.

684 probe sets from the Affymetrix exon microarray based upon ANOVA significance were evaluated for RNA biomarker signature for male subjects for differentiating IVD or IVDCs subjects from control subjects without IVD or IVDCs. The resulting RNA biomarker signature was composed of an optimal 16 probe set for male subjects using Linear Discriminant Analysis (LDA) in Limma statistical package in Bioconductor. The inclusion criterion was statistical significance using a false discovery rate (FDR) of < 0.02.

451 probe sets from the Affymetrix exon microarray based upon ANOVA significance were evaluated for RNA biomarker signature for female subjects for differentiating IVD or IVDCs subjects from control subjects without IVD or IVDCs. The resulting RNA biomarker signature was composed of an optimal 14 probe set for male subjects using Linear Discriminant Analysis (LDA) in Limma statistical package in Bioconductor. The inclusion criterion was statistical significance using a p value of < 0.001. The MisClassification LDA for the optimal 14 probe set was p < 0.001.

Figures 1 and 2 show misclassification scores for the male and female exons probe set for identifying the RNA biomarkers, respectively, depending on the number of optimized exons and for different prediction methods. Methods of data analysis comprised the Diagonal Linear Discriminant Analysis (DLDA), (regular) Linear Discriminant Analysis (LDA), Fisher Discriminant Analysis (FDA), Quadratic Discriminant Analysis (QDA), Partial Least Squares followed by Linear Discriminant Analysis (PLSLDA), Partial Least Squares followed by Random Forrest (PLSRF) or Random Forest (RF) for the classification of the microarray data.

For both the female and male exon probe set data showed that the Linear Discriminant Analysis (LDA) gave the best performance due to its sensitivity to outliers and robustness compared to the other methods. Figures 1 and 2 show that the best performance, the preferred number of optimized exons is 16 and 14 for male and female exon probe sets (for identifying the RNA biomarkers), respectively. The optimal probe sets and RNA biomarkers for female subjects and male subjects were identified and are presented in tables 3 and 6, respectively.

### References

Bastiaansen et al. TLR4 accessory molecule RP105 (CD180) regulates monocytedriven arteriogenesis in a murine hind limb ischemia model. PLoS One. 2014 Jun 19;9(6).
Bot et al. Local Mast Cell Activation Promotes Neovascularization. Cells. 2020 Mar 12;9(3):701.
Gibbons et al. in Circulation (2004) Suppl. IV: IV-47-IV-58.
Robin et al. in Journal of the American College of Cardiology, Vol. 50, No.8 (2007), pp. 727-737.
Sambrook *et al.* (1989) .
Sambrook and Russell (2001).
Smyth, G. K. Limma: linear models for microarray data. Bioinformatics and Computational Biology Solutions using R and Bioconductor. Springer, 2005, 397-420.

## Claims

1. An *in vitro* method for diagnosis of occurrence and/or assessing severity of ischemic vascular disease (IVD) and/or ischemic vascular disease-related complication (IVDC) in a female subject, said method comprising the steps of:
a. determining a first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a test sample collected from said female subject, and
b. comparing said first expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) collected from said female subject with a control expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1 from a control sample collected from control female subject(s),
c. diagnosing occurrence and/or assessing severity of IVD and/or IVDC based on the differences between the first expression level and the control expression level, wherein it is determined that IVD and/or IVDC occurred or is severe if at least two, preferably at least five, preferably at least ten, more preferably at least fourteen, RNA biomarkers from said group are different between the first expression level and the control expression level.

2. The method according to claim 1, comprising the steps of:
d. determining a second expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a further test sample collected from said subject at a later time point than the first test sample,
e. comparing the first expression level of each RNA biomarker with the second expression level of each RNA biomarker,
f. diagnosing whether the progression of IVD and/or IVDC over time in said female subject has occurred based on the difference of at least two, preferably at least five, preferably at least ten, more preferably at least fourteen, RNA biomarkers from said group between the first expression level and the second expression level in said female subject.

3. The method according to claim 1, comprising the steps of:
d. administering a treatment for IVD and/or IVDC after step c.,
e. determining a second expression level of each RNA biomarker from the group comprising ADAMTS8, AK094845, AMOT, BTRC, CCDC57, CNTNAP3, COL2A1, DCHS2, DENND5B, FOXN4, GTF2IRD1, HNRNPR, KIF18B, LPAR6, NBLA00301, NKAIN3, NRXN2, MMP11, PRSS33, PTS, QTRTD1, STK36, TBCB, TCONS_00015170-XLOC_007214, TDG and UROC1, in CD45+ mononuclear cells (MNC) from a further test sample collected from said subject at a later time point than the first test sample,
f. comparing the first expression level of each RNA biomarker with the second expression level of each RNA biomarker,
g. determining whether the treatment for IVD and/or IVDC has improved the occurrence and/or severity of IVD and/or IVDC in said female subject based on the difference of at least two, preferably at least five, preferably at least ten, more preferably at least fourteen, RNA biomarkers from said group between the first expression level and the second expression level in said female subject.

4. An *in vitro* method for diagnosis of occurrence and/or assessing severity of ischemic vascular disease (IVD) and/or ischemic vascular disease-related complication (IVDC) in a male subject, said method comprising the steps of:
a. determining a first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a test sample collected from said male subject, and
b. comparing said first expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) collected from said male subject with a control expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249 from a control sample collected from control male subject(s),
c. diagnosing occurrence and/or assessing severity of IVD and/or IVDC based on the differences between the first expression level and the control expression level, wherein it is determined that IVD and/or IVDC occurred or is severe if at least two, preferably at least ten, more preferably at least fourteen, more preferably at least sixteen, more preferably at least eighteen, RNA biomarkers from said group are different between the first expression level and the control expression level.

5. The method according to claim 4, comprising the steps of:
d. determining a second expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a further test sample collected from said subject at a later time point than the first test sample,
e. comparing the first expression level of each RNA biomarker with the second expression level of each RNA biomarker,
f. diagnosing whether the progression of IVD and/or IVDC over time in said male subject has occurred based on the difference of at least two, preferably at least ten, more preferably at least fourteen, more preferably at least sixteen, more preferably at least eighteen, RNA biomarkers from said group between the first expression level and the second expression level in said male subject.

6. The method according to claim 4, comprising the steps of:
d. administering a treatment for IVD and/or IVDC after step c.,
e. determining a second expression level of each RNA biomarker from the group comprising ENST00000440123, TCONS_00025321-XLOC_012127, MTPAP, ENST00000430988 , USP12-AS1, ProbSetID_17118935, TRH, XR_109422 // LOC100507049, ENST00000456944, TBX5 , TCONS_I2_00026656-XLOC_I2_013873, ExonID_5262586, RGL3, MFSD2B, CALD1, ENST00000518932, TOM1L2, LAMA4, COQ6, SALL4, CABLES1, POLRMT, SLC23A1, TREML2, SNHG15, CYP51A1, ExonID_5299845 and TCONS_00017554-XLOC_008249, in CD45+ mononuclear cells (MNC) from a further test sample collected from said subject at a later time point than the first test sample,
f. comparing the first expression level of each RNA biomarker with the second expression level of each RNA biomarker,
g. determining whether the treatment for IVD and/or IVDC has improved the occurrence and/or severity of IVD and/or IVDC in said male subject based on the difference of at least two, preferably at least ten, more preferably at least fourteen, more preferably at least sixteen, more preferably at least eighteen, RNA biomarkers from said group between the first expression level and the second expression level in said male subject.

7. The method according to any one of claims 1-6, wherein IVD and/or IVDC is early onset of IVD and/or early onset of IVDC in said subject.

8. The method according to any one of claims 1-7, wherein the test sample and the control sample are a biological sample selected from the group consisting of urine, serum, plasma, buffy coat, stool, saliva, tissue specimen, blood, blood cells or preferably mononuclear cells.

9. The method according to any one of claims 1-8, wherein the expression level of each RNA biomarker is determined by northern blotting, nuclease protection assay, *in situ* hybridization, DNA microarray, exon DNA microarray or quantitative RT-PCR, biochemical assays, Luminex technology, chromatographic assays, mass spectrometric assays or hybridization assays using labelled hybridization probes such as those as taught herein), RNA/DNA microarray assay, RNA/DNA Chip assays or immunoassays, ELISA assays.

10. The method according to any one of claims 1-3 and 7-9, wherein at least fourteen RNA biomarkers from the group comprising ADAMTS8, AK094845, AMOT, BTRC, CNTNAP3, COL2A1, DCHS2, DENND5B, LPAR6, MMP11, NBLA00301, PRSS33, QTRTD1 and TDG expression levels are different between the first test sample and the further or control sample.

11. The method according to any one of claims 4-9, wherein at least sixteen RNA biomarkers from the group comprising CYP51A1 , TREML2, ProbSetID_17118935, TRH, TCONS_I2_00026656-XLOC_I2_013873, SLC23A1 , TCONS_00017554-XLOC_008249, POLRMT , ENST00000518932, COQ6, ExonID_5299845, MFSD2B, SNHG15, CABLES1, RGL3 and TCONS_00025321-XLOC_012127 expression levels are different between the first test sample and the further or control sample.
